(19) Europäisches Patentamt
European Patent Office
Office européen des brevets

(11) Numéro de publication : **0 338 895 B1**

(12) # FASCICULE DE BREVET EUROPEEN

(45) Date de publication du fascicule du brevet : **23.09.92 Bulletin 92/39**

(51) Int. Cl.$^5$ : **C07D 311/58,** A61K 31/35

(21) Numéro de dépôt : **89401016.4**

(22) Date de dépôt : **13.04.89**

(54) **Nouveaux dérivés hétéroarotinoides, leurs procédés de préparation et les compositions pharmaceutiques qui les contiennent.**

(30) Priorité : **13.04.88 FR 8804870**

(43) Date de publication de la demande : **25.10.89 Bulletin 89/43**

(45) Mention de la délivrance du brevet : **23.09.92 Bulletin 92/39**

(84) Etats contractants désignés : **AT BE CH DE ES FR GB GR IT LI LU NL SE**

(56) Documents cités : **GB-A- 2 119 801**

(73) Titulaire : **ADIR ET COMPAGNIE**
**1 rue Carle Hébert**
**F-92415 Courbevoie Cédex (FR)**

(72) Inventeur : **Brion, Jean Daniel**
**76 rue du Château**
**F-95320 Saint Leu la Forêt (FR)**
Inventeur : **Le Baut, Guillaume**
**5 rue de la Baugerie**
**F-44230 Saint Sébastien sur Loire (FR)**
Inventeur : **Ducrey, Patrick**
**7 rue du Docteur Zamenhof**
**F-92500 Rueil Malmaison (FR)**
Inventeur : **Piessard-Robert, Sylvie**
**11 Bld des Martyrs Nantais**
**F-44200 Nantes (FR)**
Inventeur : **Cudennec, Claude**
**22 bis avenue de Circourt**
**F-78170 La Celle St Cloud (FR)**
Inventeur : **Seurre, Geneviève**
**3 impasse de l'Enfant Jésus**
**F-75015 Paris (FR)**

**EP 0 338 895 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés doués de propriétés anticancéreuses appartenant à la famille des rétinoïdes, leur procédé de préparation et les compositions pharmaceutiques qui les contiennent.

On connaît de nombreuses substances de la famille des rétinoïdes réputées pour leurs propriétés anticancéreuses. Les brevets US 4,054,589 ; US 4,106,681 ; US 4,137,246 ; US 4,165,103 ; US 4,169,100 ; US 4,171,318 ; US 4,224,244 ; BE 861982 ; FR 2 556 348 ; EP 111124 décrivent des dérivés de la famille des rétinoïdes dont la chaîne tétraénique est portée par une structure monocyclique cyclohexénique ou benzénique.

Plus particulièrement, le brevet US 4,105,681 décrit l'étrétinate utilisé en thérapeutique et dont les propriétés anticancéreuses sont aujourd'hui connues.

Par ailleurs, les brevets US 4,588,750, 4,326,055 et GB 2 119801 décrivent des molécules dérivées des rétinoïdes, dans lesquelles la chaîne tétraénique est remplacée par un groupement βméthyl styryl, ce dernier étant fixé sur une structure bicyclique dérivée du tétrahydronaphtalène.

Les besoins de la thérapeutique exigent le développement constant de nouveaux anticancéreux dans le double but d'obtenir des molécules à la fois plus actives mais également moins toxiques.

Les dérivés de la présente invention possèdent une structure originale constituée d'un ensemble β méthyl styrène fixé sur une structure bicyclique de type chromène, que l'on rencontre dans de nombreuses substances naturelles, éventuellement substitués sur l'un ou l'autre noyau.

L'originalité de la structure des composés de la présente invention permet l'obtention de propriétés pharmacologiques particulièrement intéressantes puisque, dans les tests étudiés supérieures à celles de l'étrétinate pour de nombreux dérivés : en outre, les composés de l'invention sont d'une toxicité nettement inférieure à celle des dérivés d'activité comparable connus jusqu'à ce jour.

Plus particulièrement la présente invention a pour objet des produits de formule générale (I) :

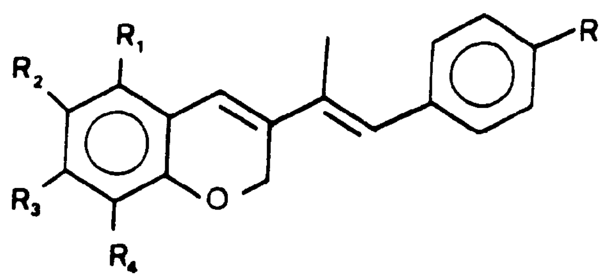

(I)

dans laquelle :

R représente un atome d'hydrogène, un atome d'halogène, ou un groupement hydroxy, alcoyle inférieur, alcoyloxy inférieur, carboxyle, alcoyloxy inférieur carbonyl, arylalcoyloxy inférieur carbonyle, aminocarbonylé, mono ou dialcoyle inférieur amino carbonyle, aryl alcoyle inférieur amino carbonyle, N hétérocycylaminocarbonyl, thio, alcoyle inférieur thio, sulfonyle, alcoyle inférieur sulfonyl,

$R_1$, $R_2$, $R_3$, $R_4$ identiques ou différents représentent un atome d'hydrogène, un atome d'halogène, un groupement alcoyle inférieur, alkényl inférieur, alcoyloxy inférieur ou alkényloxy inférieur, éventuellement substitués par un ou plusieurs atomes d'halogène,

leurs isomères, énantiomères, diastéréoisomères ainsi que lorsque R représente un carboxyle, leurs sels d'addition à une base pharmaceutiquement acceptable, et lorsque R contient un groupement basique, leurs sels d'addition à un acide pharmaceutiquement acceptable.

Les composés préférés possèdent la configuration E au niveau du groupement βméthylstyryl.

Parmi les bases susceptibles de salifier les composés de formule (I) dans lesquels R représente un groupement carboxyle, on peut citer à titre d'exemple, les hydroxydes de sodium, potassium, calcium, ou d'aluminium, les carbonates de métaux alcalins, ou alcalino terreux, ou des bases organiques comme la triéthylamine, la benzylamine, la diéthanolamine, la tert.butylamine, la dicyclohexylamine, l'arginine etc...

Parmi les acide susceptibles de salifier les composés de formule (I), on peut citer à titre d'exemple non limitatif, les acides chlorhydrique, sulfurique, tartrique, maléique, fumarique, oxalique, méthane sulfonique, camphorique etc...

Par radicaux alcoyle inférieur, alcoyloxy inférieur, alkényl inférieur, alkényloxy inférieur, on entend des groupements linéaires ou ramifiés comprenant entre 1 et 6 atomes de carbone.

Par hétérocyclyl, on entend un système mono ou bicyclique saturé ou non, incluant dans un squelette carboné un ou plusieurs hétéroatomes tels que le soufre, l'oxygène ou l'azote. Citons à titre d'exemple non limitatif,

la pyridine, le thiophène, le pyrrole, le benzothiophène, le benzopyrrole, la pyridine, la pyrimidine, la pipéridine, la morpholine, la thiomorpholine, la pyrrolidine etc...

La présente invention s'étend également à un procédé de préparation des dérivés de formule (I) caractérisé en ce que l'on condense par chauffage à reflux du solvant un dérivé de formule (II) :

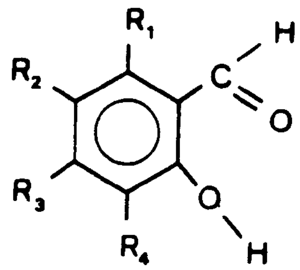

(II)

dans lequel $R_1$, $R_2$, $R_3$ et $R_4$ ont la même signification que dans la formule (I),

avec l'oxo - 3 butène - 1 de formule (III) :

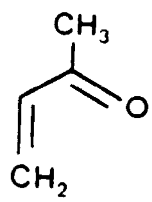

(III)

en présence d'un agent alcalin préférentiellement choisi parmi les carbonates alcalins et alcalino terreux, ou les bases organiques, comme par exemple, la triéthylamine, ou la pyridine, ou un mélange constitué d'un carbonate alcalin ou alcalino terreux et d'une base organique, dans un solvant polaire, préférentiellement choisi parmi la butanone - 2 le diméthylformamide, l'acétone, le diméthylsulfoxide,

pour conduire après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le dichlorométhane, le chloroforme, lavage de la phase organique, évaporation du solvant et purification du résidu par cristallisation, ou distillation, ou chromatographie sur colonne de silice ou d'alumine,

à un dérivé de formule (IV) :

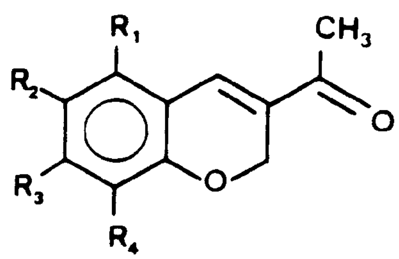

(IV)

dans laquelle $R_1$, $R_2$, $R_3$ et $R_4$ ont la même signification que dans la formule (I),

lequel, après dissolution dans un solvant organique convenable préférentiellement choisi parmi les alcools aliphatiques inférieurs est soumis, préférentiellement en présence d'un agent alcalin tel que l'hydroxyde de sodium, à hydrogénation catalytique préférentiellement par un hydrure mixte de métal alcalin tel que le borohydrure de sodium pour conduire,

après une éventuelle évaporation du milieu réactionnel, extraction par un solvant organique convenable tel que l'éther diéthylique, le chlorure de méthylène, le chloroforme, lavage puis évaporation de la phase organique et purification

à un dérivé de formule (V) :

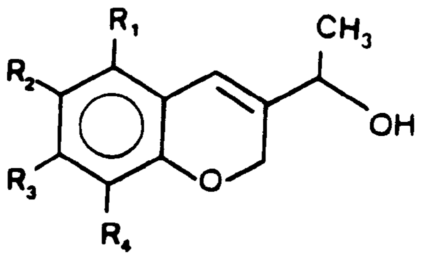

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs, à température ambiante et sous agitation pour conduire, après évaporation du solvant et purification par chromatographie sur gel de silice ou par cristallisation dans un solvant ou un mélange de solvants appropriés,

à un dérivé de formule (VI) :

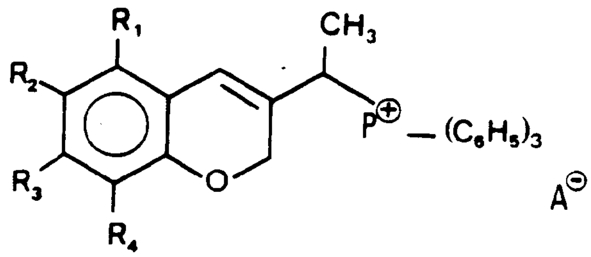

(VI)

dans laquelle $R_1$, $R_2$, $R_3$ et $R_4$ ont la même définition que dans la formule (I), et $A^-$ représente l'anion d'un hydracide,

qui, après mise en suspension dans un solvant organique, est traité par une solution de n butyllithium dans l'hexane préférentiellement à température ambiante puis traité par un dérivé de formule (VII) :

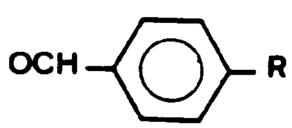

(VII)

dans laquelle R a la même signification que dans la formule (I),

préférentiellement à température ambiante, pour conduire, après dilution dans l'eau ou élimination du solvant, extraction par un solvant organique choisi parmi éther diéthylique, éther diisopropylique, chloroforme, chlorure de méthylène et purification par chromatographie sur colonne de silice à un dérivé de formule (I),

que l'on peut, si on le désire :

– soit, lorsque R représente un groupement carboxylique, salifier par une base pharmaceutiquement acceptable, ou lorsque R comporte un groupement basique, salifier par un acide pharmaceutiquement acceptable,

– soit séparer en ses isomères, diastéréoisomères, énantiomères par une technique de cristallisation ou de chromatographie puis, si on le désire, lorsque R représente un groupement carboxyle, salifier par une base pharmaceutiquement acceptable, ou lorsque R comporte un groupement basique, salifier par un acide pharmaceutiquement acceptable.

Un cas particulier des composés de formule (I) de la présente invention concerne des dérivés de formule (I/A) :

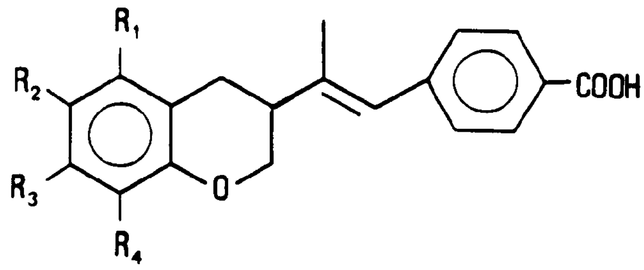

(I/A)

pour lesquels $R_1$, $R_2$, $R_3$, $R_4$ ont la même signification que précédemment.

Les dérivés de formule (I/A) peuvent être obtenus par simple hydrolyse alcaline de dérivés de formule (I/B) :

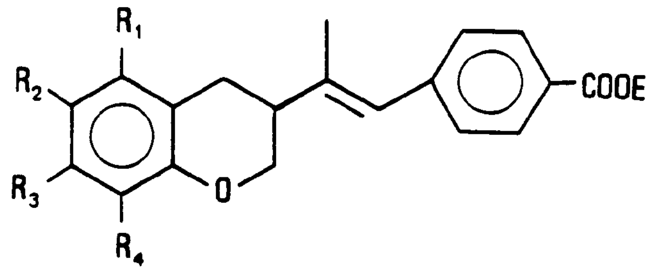

(I/B)

cas particulier des dérivés de formule (I) pour lesquels R représente un groupement COOE dans laquelle E représente un groupement alkyle inférieur et $R_1$, $R_2$, $R_3$ et $R_4$ ont la même signification que précédemment,

suivie le cas échéant de la séparation des isomères, énantiomères, diastéréoisomères et éventuellement de la salification par une base pharmaceutiquement acceptable.

Les composés de formule (I) possèdent des propriétés pharmacologiques très intéressantes. Ils inhibent de façon plus importante que l'étrétinate la croissance de la lignée L 1210 chez la souris, et, fait particulièrement intéressant pour leur utilisation en thérapeutique, ne semblent pas entraîner de toxicité hypervitaminique A, induite par l'étrétinate, et dont les symptômes sont la perte de poids rapide, l'alopécie et la fragilisation des os.

Les composés selon la présente invention trouvent donc une utilisation en thérapeutique en tant qu'agents antitumoraux, pour le traitement ou la prophylaxie des néoplasmes bénins ou malins, ainsi que dans les indications classiques des rétinoïdes, tels que les désordres cutanés (acné, psoriasis), ainsi que les troubles dégénératifs et/ou inflammation des muqueuses.

La présente invention a également pour objet les compositions pharmaceutiques contenant les produits de formule (I), ou un de leurs sels d'addition à une base ou un acide pharmaceutiquement acceptable lorsque R représente un groupement salifiable, seul ou en combinaison avec un ou plusieurs excipients ou véhicules inertes, non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire et notamment les comprimés, simples ou dragéifiés, les comprimés sublinguaux, les sachets, les paquets, les gélules, les glossettes, les tablettes, les suppositoires, les crèmes, pommades, gels dermiques, les préparations injectables ou buvables, les aérosols, les gouttes oculaires ou nasales...etc...

La posologie utile varie selon l'âge et le poids du patient, la voie d'administration, la nature de l'indication thérapeutique et des traitements éventuellement associés et s'échelonne entre 0,1 et 200 mg par jour.

Les exemples suivants illustrent l'invention mais ne la limitent en aucune façon.

Les spectres de résonance magnétique nucléaire, sauf indication contraire, sont réalisés dans le chloroforme avec un appareil à 60 MHz, en utilisant le TMS comme référence interne. Les déplacements sont exprimés en ppm.

Les préparations figurant ci-dessous concernent des dérivés qui ne font pas partie de la présente invention, mais qui sont utiles en tant qu'intermédiaires au cour de la synthèse des dérivés de l'invention.

**PREPARATION 1 :**

**FORMYL - 4 BENZOATE D'ETHYLE**

**STADE A : ACIDE HYDROXYMETHYL - 4 BENZOIQUE**

A une solution de 13,86 g (92,3 mmole) d'acide formyl - 4 benzoïque dans 200 ml d'éthanol absolu, est ajoutée sous agitation goutte à goutte une solution de 3,68 g (92,3 mmole) de borohydrure de sodium, dissous dans 30 ml d'eau additionnés de 5 ml de NaOH 5N. Le milieu réactionnel est laissé ainsi 5 heures sous agitation à température ambiante. L'éthanol est alors éliminé par évaporation sous vide et le résidu est repris par de l'eau, puis acidifié par de l'acide chlorhydrique 5N : le précipité obtenu est séparé par filtration sur fritté.

D'une part, le filtrat est extrait par de l'éther éthylique (3 x 150 ml), d'autre part, le précipité est épuisé par de l'éther éthylique à chaud. L'évaporation des phases organiques livre 10,5 g du produit attendu

Rendement global : 75 %

Point de fusion : 178 °C.

**STADE B : HYDROXYMETHYL - 4 BENZOATE D'ETHYLE**

4 g (26,3 mmole) d'acide hydroxyméthyl - 4 benzoïque dans 1000 ml d'éthanol absolu sont chauffés au reflux du solvant pendant 15 heures en présence d'environ 1 g d'acide sulfurique concentré. L'alcool éthylique est alors évaporé et le résidu repris à l'eau. Après extraction de la phase aqueuse par 3 x 100 ml d'éther éthylique, les phases organiques sont lavées jusqu'à neutralité par une solution saturée d'hydrogénocarbonate de sodium. Après élimination du solvant, il reste 4,56 g d'une huile jaune qui ne tarde pas à cristalliser.

Rendement global : 96 %

**STADE C : FORMYL - 4 BENZOATE D'ETHYLE**

A une solution de 2,5 g (13,9 mmole) d'hydroxyméthyl - 4 benzoate d'éthyle dans un mélange n - hexane - tétrachlorure de carbone (50 ml - 30 ml), on ajoute sous agitation et à température ambiante, 24,1 g (0,277 mmole) de dioxyde de manganèse. Après une demi-heure d'agitation, la réaction est complète. Après filtration du milieu réactionnel et élimination des solvants sous vide, sont obtenus 1,76 g d'une huile jaune, monotâche en CCM. Une cristallisation dans l'éther isopropylique livre un solide blanc.

Rendement global : 71 %

Point de fusion : 157 °C.

**PREPARATION 2 :**

**FORMYL - 4 BENZOATE DE METHYLE**

En remplaçant dans la préparation 1, stade B l'éthanol par le méthanol, on obtient le produit attendu.

**PREPARATION 3 :**

**N - ETHYL FORMYL - 4 BENZAMIDE**

Ce composé est obtenu en 3 stades à partir de l'acide formyl - 4 benzoïque lequel est soumis à l'action du chlorure de thionyle pour conduire au chlorure d'acide correspondant, lequel est traité par l'éthylamine dans un solvant organique approprié, comme le benzène par exemple, pour conduire au produit attendu.

Rendement global : 25 %

Point de fusion : 100°C

**PREPARATION 4 :**

**ETHYL FORMYL - 4 PHENYL SULFONE**

Ce composé est obtenu en 4 étapes à partir du para bromothiophénol lequel est soumis à l'action d'un agent d'éthylation comme l'iodure d'éthyle en présence d'éthylate de sodium pour conduire au bromo - 4S éthylthiophénol, lequel est traité par le magnésium dans un solvant comme le tétrahydrofuranne, l'éther éthylique

ou diisopropylique et soumis à l'action d'un acide pour conduire à la para éthylthio benzaldéhyde qui sous l'action d'oxydants permet l'obtention du produit attendu.

Point de fusion : 114 °C

**PREPARATION 5 :**

**DIMETHYL - 5,6 HYDROXY - 2 METHOXY - 4 BENZALDEHYDE**

**STADE A : DIACETATE DE (DIMETHYL - 2,3 PHENYL) - 1,5**

Dans un ballon équipé d'un réfrigérant et d'une arrivée d'azote, sont introduits 27 g (0,192 mmole) de dimédone, 300 ml d'anhydride acétique, puis goutte à goutte et sous agitation 12 ml d'acide sulfurique concentré, un net échauffement du milieu réactionnel est observé. La réaction est ensuite chauffée au reflux pendant une heure sous azote. Après refroidissement, le milieu réactionnel est jeté sur un mélange glace-eau et agité ainsi 40 minutes. La phase aqueuse est alors extraite par 4 x 250 ml d'éther. Après neutralisation par une solution saturée d'hydrogénocarbonate de sodium et lavage par solution saturée de saumure, la phase éthérée est séchée sur sulfate de sodium. L'élimination du solvant livre un résidu huileux (21 g) qui est distillé sous vide.

Rendement : 49 %.

**STADE B : DIMETHYL - 2,3 HYDROXY - 5 PHENOL**

(M.S. KABLAOUI, J. ORG. CHEM. 1974, 39 (25) 3696)

13,30 g (60 mmole) de diacétate de (diméthyl - 2,3 phényl) - 1,5 sont saponifiés après 2 heures de chauffage au reflux par une solution d'hydroxyde de sodium 6 g ((0,15 mmole) de NaOH dans 50 ml d'eau). Après refroidissement et acidification de la solution par de l'acide chlorhydrique 2,5 N, le milieu réactionnel est extrait par 3 x 250 ml d'éther éthylique. La phase organique est lavée à l'eau, puis l'éther éthylique éliminé. Le résidu huileux cristallise. Après reprise des cristaux par le benzène et séparation par filtration sur fritté, sont obtenus 4,45 g d'un solide blanc.

Rendement : 53 %

Point de fusion :135 - 136 °C.

**STADE C : DIHYDROXY - 2,4 DIMETHYL - 5,6 BENZALDEHYDE**

(A. ROBERTSON, W.B. WHALLEY, J. CHEM. SOC., 1949, 3033)

Dans un ballon équipé d'une entrée de gaz, d'un thermomètre et d'un réfrigérant (les gaz sortants étant piégés par 3 barboteurs successifs : $H_2SO_4$, solution de $KMnO_4$ saturée, NaOH 3N), 4,450 g (32 mmole) de diméthyl - 2,3 hydroxy - 5 phénol sont dissous dans 100 ml d'éther éthylique anhydre. Après avoir additionné 5,635 g (48 mmole) de cyanure de zinc, on fait passer un courant d'acide chlorhydrique gazeux pendant 2 heures sous agitation et à température ambiante. On précipité abondant apparaît au fur et à mesure du temps. En fin d'opération, le milieu réactionnel est traversé par un fort courant d'azote. L'insoluble est séparé par filtration et dissous dans de l'eau bouillante. Après refroidissement l'aldéhyde précipite. Après filtration, les cristaux sont lavés à l'eau et séchés.

Rendement : 71 %

Point de fusion : 196 °C.

**STADE D : DIMETHYL - 5,6 HYDROXY - 2 METHOXY - 4 BENZALDEHYDE**

Dans un ballon, 23 g (0,138 mmole) de la dihydroxy - 2,4 diméthyl - 5,6 benzaldéhyde sont dissous dans 200 ml d'acétone anhydre. A la solution sont ajoutés successivement 38 g (0,276 mmole) de carbonate de potassium sec et 9,50 ml (0,152 mmole) d'iodure de méthyle. Le milieu réactionnel est chauffé à reflux pendant 20 heures. Après refroidissement et filtration de l'insoluble, l'acétone est éliminée par évaporation sous vide. Le résidu est repris à l'eau et extrait par 3 x 150 ml d'éther éthylique puis par 2 x 100 ml de dichlorométhane. Après évaporation des solvants organiques. La diméthyl - 5,6 hydroxy - 2 méthoxy - 4 benzaldéhyde cristallise. La purification après chromatographie sur colonne de gel silice, livre 24,8 g.

Rendement : 83 %

Point de fusion : 121 °C.

**EXEMPLE 1 : (PHENYL - 1 PROPENYL - 2) - 3 2H - CHROMENE**

**STADE A : ACETYL - 3 2H - CHROMENE**

A une suspension de 0,14 g (1 mmole) de carbonate de potassium dans 50 ml de butanone - 2, additionner, à température ambiante 1,22 g (10 mmole) d'aldéhyde salicylique ainsi que 0,66 g (10 mmole) d'oxo - 3 butène - 1. Porter à reflux sous agitation et maintenir cette température pendant 4 heures. Evaporer le milieu réactionnel au bain-marie sous vide et reprendre le résidu par 100 ml d'eau et extraire à trois reprises par 75 ml d'éther diéthylique. Réunir les phases organiques, laver jusqu'à neutralité par une solution aqueuse saturée de chlorure de sodium, sécher sur sulfate de sodium, filtrer et évaporer au bain-marie sous vide, et recristalliser dans l'hexane.

Rendement : 57 %.

**STADE B : (HYDROXY - 1 ETHYL) - 3 2H - CHROMENE**

Dissoudre 1,75 g (10 mmole) d'acétyl - 3 2H chromène obtenu au stade précédent, dans 50 ml d'éthanol. Additionner, en une fois, sous agitation magnétique, une solution de 0,38 g (10 mmole) de borohydrure de sodium dans 5 ml d'une solution aqueuse d'hydroxyde de sodium. Maintenir l'agitation pendant 2 heures puis évaporer le mélange solvant. Reprendre le résidu par 50 ml d'eau et extraire à trois reprises par 50 ml d'éther diéthylique. Laver la solution éthérée par une solution aqueuse saturée de chlorure de sodium jusqu'à neutralité des eaux de lavage, sécher et évaporer la phase organique., et utiliser le produit ainsi obtenu dans la phase suivante.

Rendement : 95 %.

**STADE C : BROMURE DE [(2H - CHROMENYL - 3) - 1 ETHYL] TRIPHENYL PHOSPHONIUM**

Placer sous atmosphère d'azote à température ambiante 1,77 g (10 mmole) d'(hydroxy - 1 éthyl) - 3 2H - chromène obtenu au stade précédent, et 3,43 g (10 mmole) de bromhydrate de triphénylphosphonium dans 80 ml de méthanol. Agiter 96 heures. Eliminer le méthanol par évaporation et chromatographier le résidu obtenu sur colonne de gel de silice (70 - 230 Mesh) en utilisant comme solvant d'élution le mélange chlorure de méthylène éthanol (95/5/v/v), et cristalliser dans le mélange solvant benzène acétone.

Rendement : 85 %

Point de fusion : 198°C.

**STADE D :** (Phényl - 1 propényl - 2) - 3 2H - chromène

Placer 5 g (10 mmole) de bromure de [(2H - chroményl - 3) - 1 méthyl] triphényl phosphonium obtenu au stade précédent, en suspension dans 30 ml de tétrahydrofuranne. Ajouter à température ambiante sous atmosphère d'azote et sous agitation magnétique 6,9 ml d'une solution 1, 6M de n butyllithium dans l'hexane. Laisser en contact dix minutes puis additionner en 20 minutes une solution de 1,06 g d'aldéhyde benzoïque (10 mmole) dans 50 ml de TMF. Poursuivre l'agitation pendant 4 heures. Diluer le milieu réactionnel avec 75 ml d'eau et extraire à 3 reprises par 75 ml d'éther diéthylique. Réunir les phases organiques, laver jusqu'à neutralité par une solution aqueuse saturée de chlorure de sodium. Sécher et évaporer la phase organique, purifier le résidu par deux chromatographies préparatives sur colonne de gel de silice.

1/ $SiO_2$ 70 - 230 Mesh

2/ $SiO_2$ 200 - 400 Mesh

On obtient ainsi une huile, mélange d'isomères Z et E purifiés par cirstallisation fractionnée.

Rendement : 70 %

Point de fusion : huile

Analyse élémentaire :

Calculée : C : 87,06 H : 6,49

Trouvée : C : 86,84 H : 6,36

Caractéristiques spectrales :

Infrarouge ($cm^{-1}$) : 1600, 1580, 1480, 1460, 1220, 750, 700

Résonance magnétique nucléaire (60 MHz) :

$\delta$ = 2,05 et 2,15 3H singulet $CH_3$

$\delta$ = 4,85 et 5,20 2H singulet $CH_2$ chromène

$\delta$ = 6,45 - 7,60 massif 11H éthyléniques et aromatiques.

**EXEMPLE 2 : (PHENYL - 1 PROPENYL - 2) - 3 2H - CHROMENE ISOMERE E**

Procéder comme dans l'exemple 1, et séparer l'isomère E du mélange d'isomères obtenus dans l'exemple 1, Stade D.

Rendement : 10 %

Analyse élémentaire :

Calculée : C : 87,06 H : 6,49
trouvée : C : 86,30 H : 6,50

Caractéristiques spectrales :

Infrarouge : 1600, 1580, 1480, 1460, 1220, 750, 700

Résonance magnétique nucléaire Solvant $CDCl_3$ :

$\delta$ = 2,15 ppm 3H singulet $CH_3$
$\delta$ = 5,20 ppm 2H singulet $CH_2$ chromène
$\delta$ = 6,45 à 7,60 ppm 11H éthylènique et aromatique.

**EXEMPLES 3 A 19 :**

En procédant comme dans les exemples 1 ou 2, selon que l'on désire ou non séparer l'isomère (E) du mélange on obtient :

. en remplaçant au stade D l'aldéhyde benzoïque par l'ester méthylique de l'acide formyl - 4 benzoïque (obtenu dans la préparation 2), on obtient des [(2H - chroményl - 3) - 2 propényl] - 4 benzoates de méthyle.

. en remplaçant au stade D, l'aldéhyde benzoïque par l'ester éthylique de l'acide formyl - 4 benzoïque (obtenu dans la préparation 1), on obtient des [(2H - chroményl - 3) - 2 propényl] - 4 benzoates d'éthyle.

. en remplaçant au stade D, l'aldéhyde benzoïque par le N - éthyl formyl - 4 benzamide (obtenu dans la préparation 3), on obtient des N - éthyl [(2H - chroményl - 3) - 2 propényl] - 4 benzamides.

. en remplaçant au stade D, l'aldéhyde benzoïque par l'éthyl formyl - 4 phényl sulfone (obtenu dans la préparation 4), on obtient des éthyl [(2H - chroményl - 3) - 2 propényl] - 4 phényl sulfones.

Il est également possible de procéder comme dans les exemples 1 et 2 en remplaçant :

* au stade A de l'exemple 1 l'aldéhyde salicylique par un aldéhyde salicylique substitué de formule générale (II/1)

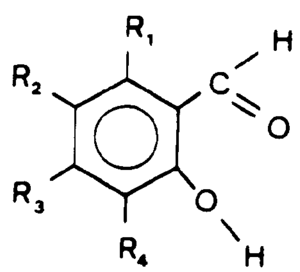

(II/1)

cas particulier des dérivés de formule (II) dans laquelle $R_1$, $R_2$, $R_3$, $R_4$ ont la même signification que dans la formule (I) à l'exclusion du cas où $R_1$, $R_2$, $R_3$, $R_4$ simultanément représentent un atome d'hydrogène.

* au stade A, de l'exemple 1, l'aldéhyde salicylique par un dérivé de formule (II/1) et au stade D l'aldéhyde benzoïque par :

– soit l'ester méthylique de l'acide formyl - 4 benzoïque,
– soit l'ester éthylique de l'acide formyl - 4 benzoïque,
– soit le N éthyl formyl - 4 benzamide,
– soit l'éthyl formyl - 4 phényl sulfone.

Les tableaux A visualisent les données relatives à la synthèse des exemples 3 à 19.

Le tableau A1 indique plus particulièrement les caractéristiques des acétyl - 3 chromène (dérivés de formule (IV)).

Le tableau B visualise les caractéristiques physicochimiques des exemples 3 à 19.

TABLEAU A -

| EX N° | $R_1$ | $R_2$ | $R_3$ | $R_4$ | R | Stéréo-chimie | STADE A | | STADE B | STADE C | | STADE D | |
|---|---|---|---|---|---|---|---|---|---|---|---|---|---|
| | | | | | | | T1 | Rdt (%) | Rdt (%) | Rdt (%) | F (°C)* | T2 (n) | Rdt (%) |
| 3 | H | Cl | H | $CH_3$ | H | E + Z | 4 | 50 | 93 | 58 | 172 | 6 | 75 |
| 4 | H | $CH_3$ | H | $CH_3$ | H | E + Z | 1,5 | 81 | 86 | 68 | 164 | 6 | 48 |
| 5 | $CH_3$ | $CH_3$ | $OCH_3$ | H | H | E | 1,5 | 25 | | 57 | 134 | 6 | 12 |
| 6 | H | H | H | H | $COOCH_3$ | E | 4 | 57 | 95 | 85 | 198 | 24 | 22 |
| 7 | H | Cl | H | $CH_3$ | $COOCH_3$ | E | 4 | 50 | 93 | 58 | 172 | 24 | 20 |
| 8 | H | Cl | H | Cl | $COOCH_3$ | E | 4 | 20 | 87 | 46 | 193 | 24 | 18 |
| 9 | $CH_3$ | $CH_3$ | $OCH_3$ | H | $COOCH_3$ | E | 1,5 | 25 | | 57 | 134 | 18 | 30 |
| 10 | H | H | H | H | $COOC_2H_5$ | E | 4 | 57 | 95 | 85 | 198 | 24 | 24 |
| 11 | H | Cl | H | $CH_3$ | $COOC_2H_5$ | E | 4 | 50 | 93 | 58 | 172 | 24 | 16 |
| 12 | H | $CH_3$ | H | $CH_3$ | $COOC_2H_5$ | E + Z | 1,5 | 81 | 86 | 68 | 164 | 18 | 70 |
| 13 | H | H | H | H | $CONHC_2H_5$ | E | 4 | 57 | 95 | 85 | 198 | 18 | 23 |
| 14 | H | Cl | H | $CH_3$ | $CONHC_2H_5$ | E | 4 | 50 | 93 | 58 | 172 | 18 | 42 |
| 15 | H | $CH_3$ | H | $CH_3$ | $CONHC_2H_5$ | E | 1,5 | 81 | 86 | 68 | 164 | 18 | 56 |
| 16 | H | H | H | H | $SO_2C_2H_5$ | E | 4 | 57 | 95 | 85 | 198 | 84 | 14 |
| 17 | H | Cl | H | $CH_3$ | $SO_2C_2H_5$ | E + Z | 4 | 57 | 93 | 58 | 172 | 6 | 41 |
| 18 | H | $CH_3$ | H | $CH_3$ | $SO_2C_2H_5$ | E + Z | 1,5 | 81 | 86 | 68 | 164 | 4 | 36 |
| 19 | H | Cl | H | Cl | $SO_2C_2H_5$ | E | 4 | 50 | 93 | 46 | 193 | 24 | 14 |

* Point de fusion du sel de phosphonium obtenu à l'issue du stade C
T1 = temps de reflux
T2 = temps de réaction.

TABLEAU A1

PURIFICATION DES ACETYL - 3 2H - CHROMENE

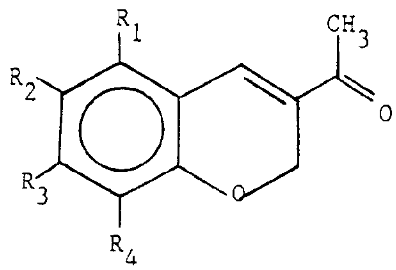

| $R_1$ | $R_2$ | $R_3$ | $R_4$ | F°C | MODE DE PURIFICATION |
|---|---|---|---|---|---|
| H | H | H | H | 58-60 | recrist. (hexane) |
| H | $OCH_3$ | H | H | 59 | recrist. (éther isopropylique) |
| H | H | $OCH_3$ | H | 72 (jaune) | chrom. sur colonne de gel de silice (200-400 mesh); éluant : $CH_2Cl_2$ |
| H | $CH_3$ | H | $CH_3$ | 41-42 (jaune) | chrom. sur colonne de gel de silice (70-230 mesh); éluant : $CH_2Cl_2$ |
| H | Cl | H | $CH_3$ | 78-79 (jaune) | chrom. sur colonne de gel de silice (70-230 mesh); éluant : $CH_2Cl_2$ puis recrist. (éther isopropylique- éther de pétrole) |
| H | Cl | H | Cl | 108 (jaune) | chrom. sur colonne de gel de silice (70-230 mesh); éluant : $CH_2Cl_2$ |
| $CH_3$ | $CH_3$ | $OCH_3$ | H | 110 (jaune) | chrom. sur colonne d'alumine et recrist. dans l'éthanol |

TABLEAU B - CARACTERISTIQUES PHYSICOCHIMIQUES DES EXEMPLES 3 A 18

| Ex N° | F (°C) | ANALYSE ELEMENTAIRE | | SPECTROMETRIE | |
|---|---|---|---|---|---|
| | | CALCULEE | TROUVEE | INFRA-ROUGE (cm-1) | RESONANCE MAGNETIQUE NUCLEAIRE * (δ) ppm |
| 3 | Huile | C 76,89<br>H 5,77 | C 76,91<br>H 5,80 | 1600, 1580, 1460 1200<br>750, 690 | 2,15 : s; 3H; $CH_3$ (chromène)<br>2,25; s; 3H; $CH_3$ (éthyline)<br>5,20 : s.e.; 2H; chromène-6,65. s, 1H éthylène<br>6,85-7,55 : 8H : aromatiques + 1H chromène |
| 4 | Huile | | | | |
| 5 | 97°C | | | | |
| 6 | 115°C | C 78,43<br>H 5,88 | C 78,33<br>H 6,01 | 1720, 1610, 1120<br>760 | 2,25 : s : 3H : $CH_3$ (éthylène).4,00 s; 3H; ester<br>5,20; s.e. : 2H chromène; 6,60:s.e.; 1H éthylène<br>6,65-7,35;m ; 5H; ar et chromène<br>7,85-8,30; 4H; ar (AA'BB') |
| 7 | 135°C | C 69,32<br>H 5,54 | C 69,28<br>H 5,50 | 1725, 1610, 1155<br>760 | 2,15 : s : 3H : CH3 (éthylène)<br>2,20 s; 3H : $CH_3$:chromène<br>3,90 : s : 3H : $CH_3$ (ester)<br>5,10 s.e; 2H; $CH_2$ chromène<br>6,45; s.e : 1H; H éthylénique;<br>6,55; s.e.; 1H; $H_4$ chromène<br>7,20-8,10; 4H : AA'BB' ar et 2H ar |

s : singulet ; s.e. : signal élargi ; m : multiplet ; ar : aromatiques

TABLEAU B - (suite I)

| Ex N° | F (°C) | ANALYSE ELEMENTAIRE | | SPECTROMETRIE | |
|---|---|---|---|---|---|
| | | CALCULEE | TROUVEE | INFRA-ROUGE ($cm^{-1}$) | RESONANCE MAGNETIQUE NUCLEAIRE *(δ) ppm |
| 8 | 172°C | C 64,01<br>H 4,30 | C 63,46<br>H 4,49 | 1730, 1610, 1120 | 2,15; s : 3H; $CH_3$ (éthylène)-3,95;s : 3H (ester)<br>5,25 : s.e.; 2H; CH2 (chromène);<br>6,65-7,05 (3H, m $H_4$, $H_5$ et H éthylène)<br>7,40-8,10; 5H : AA'BB' et $H_7$ |
| 9 | 166°C | C 75,80<br>H 6,64 | C 75,36<br>H 6,68 | 1710, 1590, 1280 | 2,15 s : 3H; $CH_3$ (éthylène);<br>2,20 et 2,35; 2s; 2 x $CH_3$ (chromène)<br>3,85 s; 3H; O$\underline{CH_3}$; 3,95; s;3H; $CH_3$ ester<br>5,05; s.e.; 2H; $H_2$; chromène<br>6,35-8,25 (7H, massif, 4H arom, 2H chromère 1H<br>éthylénique |
| 10 | 120°C | C 78,73<br>H 6,29 | C 78,68<br>H 6,18 | 1730, 1620 | 1,41; t; 3H; $CH_2$-$\underline{CH_3}$; 2,15. s.e. 3H; $CH_3$ éthylène<br>4,35;q; 2H; O$\underline{CH_2}$-$CH_3$; 5,1.s.e.; 2H; $CH_2$; chromène<br>6,45; 1H; s.e.; 2H; H éthylène;<br>6,6-8,10 m; 9H; aromatiques t 1H chromène |
| 11 | 127°C | C 70,49<br>H 5,63 | C 70,42<br>H 5,10 | | 1,40; t; 3H-$CH_2$-$\underline{CH_3}$; 2,10; s; 3H; $CH_3$ chromène<br>2,20; s.e.; 3H; $CH_3$ éthylène<br>4,40; q; 2H; COO$\underline{CH_2}$$CH_3$ |

s = singulet

TABLEAU B - (suite II)

| Ex N° | F (°C) | ANALYSE ELEMENTAIRE CALCULEE | ANALYSE ELEMENTAIRE TROUVEE | SPECTROMETRIE RESONANCE MAGNETIQUE NUCLEAIRE *(δ) ppm |
|---|---|---|---|---|
| 11 suite | | | | 5,15; s.e.; 2H; $H_2$ chromène, 6,50 : s.e.; 1H éthylène<br>6,60-8,20; massif : 7H; H aromatiques + chromène |
| 12 | | C 79,28<br>H 6,94 | C 79,01<br>H 6,83 | 1,50 et 1,70; 3H ; 2t; $COOCH_2CH_3$<br>2,15 et 2,45; 9H multiplet $CH_3$ éthyl et $2CH_3$ chromène<br>4,50; 2H; q; $COO\underline{CH_2}-CH_3$<br>4,90 et 5,20; 2H; 2 s.e.; $H_2$<br>6,60 et 6,75; 1H; 2 s.e.; H éthyl<br>6,85-8,30; 7H; multiplet $H_4$, $H_5$, $H_7$ ET 4H ar |
| 13 | 187°C | C 78,97<br>H 6,63<br>N 4,38 | C 78,82<br>H 6,60<br>N 4,26 | 1,25; (t); 3H; $NH-CH_2-\underline{CH_3}$; J=7Hz; 2,15 s.e.; 3H; $CH_3$<br>3,50 ; q 2H; $NH-\underline{CH_2}-CH_3$; J=7Hz; 5,15 s.e.; 2H $H_2$<br>6,10-7,95 massif;11H;H éthylène, $H_4$, $H_5$, $H_6$, $H_7$, $H_8$, A ar AA'BB', NH |
| 14 | 166°C | C 71,83<br>H 6,03<br>N 3,81 | C 71,75<br>H 6,11<br>N 3,85 | 1,25; t 3H; $NH-CH_2-\underline{CH_3}$<br>2,15 s.e; 3H $CH_3$; éthylène<br>2,25; s; 3H; $CH_3$ (8) chromène; 3,55; q; 2H; $NH-\underline{CH_2}-CH_3$<br>5,15; s.e.; 2H; H-2 chromène, 6,20 mult.; 1H; <u>NH</u><br>6,45-8,00; 8H; m; H éthylène + H chromène + H aromatique |

s = singulet

TABLEAU B - (suite III)

| Ex N° | F (°C) | ANALYSE ELEMENTAIRE CALCULEE | ANALYSE ELEMENTAIRE TROUVEE | SPECTROMETRIE RESONANCE MAGNETIQUE NUCLEAIRE *(δ) ppm |
|---|---|---|---|---|
| 15 | 152°C | C 79,51<br>N 7,29<br>H 4,03 | C 79,47<br>H 7,37<br>N 4,06 | 1,30; t; 3H; NH-$CH_2$-<u>$CH_3$</u>; 2,15 s.e. 3H; $CH_3$ éthylène<br>2,25, 2,30:2s 2 x 3H ($CH_3$ et $CH_3$ chromène)<br>6,25 m; 1H; <u>NH</u>,6,50-8,15 (m, 8H; H éthylène + H chromène + H arom) |
| 16 | 149°C | C 70,56<br>H 5,92 | C 70,64<br>H 5,96 | Solvant DMSO d6<br>1,10; t; 3H; $CH_3$ ($C_2H_5$) J = $7H_2$-2,20 s; 3H; $CH_3$; éthyl<br>3,30; q; 2H; $CH_2$ ($C_2H_5$); J = $7H_2$-5,15 s.e.; 2H; $H_2$ chromène<br>6,50-8,10 m; 10 H; éthyl et aromatique |
| 17 | | C 64,85<br>H 5,44 | C 64,12<br>H 5,80 | 1,25 et 1,75; t; 3H; $SO_2CH_2$<u>$CH_3$</u><br>2,10; s.e.; 3H; $CH_3$ (éthylénique)<br>2,25; s; 3H; $CH_3$ (chroményl)<br>3,15; q; 2H; $SO_2CH_2CH_3$; 4,90 et 5,15; 2 s.e.; 2H; $H_2$<br>6,40-8,20; multiplet; 8H; H éthyl et aromatiques |
| 18 | | C 70,00<br>H 6,67 | C 70,28<br>H 6,23 | 1,25 et 1,85; 2t; 3H $SO_2CH_2CH_3$<br>2,15 : s.e.; 3H $CH_3$ (éthylénique)<br>2,20 : s; 6H; $2CH_3$ (chroménique)<br>3,10 : q; 2H; $SO_2$-$CH_2$-$CH_3$; 4,80-5,10 : 2H, 2s.e.; $H_2$<br>6,40-8,10 : 8H, m, H éthylène et 4H ar et $H_4$, $H_5$, $H_7$ |

s = singulet

**EXEMPLE 20 : (PHENYL - 1 PROPENYL - 2) - 3 METHOXY - 6 2H CHROMENE**

**STADE A : METHOXY - 6 ACETYL - 3 2H - CHROMENE**

A température ambiante et sous agitation magnétique ajouter 1,52 g (10 mmole) d'aldéhyde hydroxy - 2 méthoxy - 5 benzoïque, 0,66 g d'oxo - 3 butène - 1 et 1,4 g (10 mmole) de carbonate de potassium à 40 ml de butanone - 2. Porter à reflux sous agitation et maintenir à cette température pendant 2 heures. Après refroidissement verser le milieu réactionnel sur 100 ml d'eau et extraire à 3 reprises par 100 ml d'éther diéthylique. Réunir les phases organiques, laver jusqu'à neutralité par une solution aqueuse saturée de chlorure de sodium, sécher sur sulfate de sodium, évaporer la phase organique au bain-marie sous vide, et recristalliser.

Rendement : 77 %

**STADES B A D :**

Procéder comme dans l'exemple 1, en remplaçant l'acétyl - 3 2H - chromène par le méthoxy - 6 acétyl - 3 2H chromène.

**EXEMPLES 21 A 24 :**

En procédant comme dans l'exemple 20, en remplaçant au stade D l'adéhyde benzoïque par :

– l'ester méthylique de l'acide formyl - 4 benzoïque, on obtient le [(méthoxy - 6 2H - chroményl - 3) - 2 propényl] - 4 benzoate de méthyle. **(EXEMPLE 21)**

– l'ester éthylique de l'acide formyl - 4 benzoïque, on obtient le [(méthoxy - 6 2H - chroményl - 3) -2 propényl] - 4 benzoate d'éthyle. **(EXEMPLE 22)**

– le N - éthyl formyl - 4 benzamide, on obtient le N - éthyl [(méthoxy - 6 2H - chroményl - 3) - 2 propényl] - 4 benzamide. **(EXEMPLE 23)**

– l'éthyl formyl- 4 phényl sulfone, on obtient l'éthyl [(méthoxy - 6 2H - chroményl - 3) - 2 propényl] - 4 phényl sulfone. **(EXEMPLE 24)**

Le tableau C visualise les données relatives à la synthèse des 4 exemples.

Le tableau D visualise les caractéristiques physico-chimiques des produits.

TABLEAU C

SYNTHESE DES EXEMPLES 21 A 24

| EX N° | R | STEREO-CHIMIE | STADE B Rdt | STADE C | | STADE D | |
|---|---|---|---|---|---|---|---|
| | | | | Rdt (%) | F (°C) * | TR (H) | RdT (%) |
| 21 | $COOCH_3$ | E | 95 | 77 | 110 | 24 | 27 |
| 22 | $COOC_2H_5$ | E | 95 | 77 | 110 | 24 | 42 |
| 23 | $CONHC_2H_5$ | E | 95 | 77 | 110 | 18 | 38 |
| 24 | $SO_2C_2H_5$ | E | 95 | 77 | 110 | 24 | 15 |

TR = Temps de réaction

TABLEAU D : CARACTERISTIQUES PHYSICO-CHIMIQUES DES EXEMPLES 21 A 24

| Ex N° | F (°C) | ANALYSE ELEMENTAIRE | | SPECTROMETRIE |
|---|---|---|---|---|
| | | CALCULEE | TROUVEE | RESONANCE MAGNETIQUE NUCLEAIRE *(δ) Rpm ppm |
| 21 | 110°C | C 74,98<br>H 5,99 | C 74,44<br>H 6,01 | 2,10 : s, 3H (CH3, éthylénique) 3,65 s, 3H, OCH3 en 6<br>3,85 s, 3H CH3 ester 4,95 s, 2H, CH2 (2)<br>6,40 s H éthylénique<br>6,60 - 8,20 m, 8H, aromatiques et H4, H5, H7, H8 |
| 22 | Huile | C 75,41<br>H 6,33 | C 74,79<br>H 6,37 | 1,40, t, 3H OCH2 - CH3 2,15:s, 2H, CH3 éthylénique<br>3,85 s, 3H, OCH3 en 6 4,40:q, 2H, OCH2 CH3<br>4,85 s, 2H, H2 en 2<br>6,55 - 8,15 m, 9H, aromatiques et H4, H5, H8 éthylénique |
| 23 | 170°C | C 75,62<br>H 6,63<br>N 4,00 | C 75,59<br>H 6,65<br>N 4,00 | 1,25:t 3H, NHCH2CH3 J = 7H2<br>2,10:s, 3H, CH3<br>3,5:2H NHCH2CH3 J = 7H2<br>3,8 s, 3H, OCH3, 5,05:s, 2H, CH2 en 2<br>6,40 - 6,80:m, 6H, éthylénique, NH, H4, H5, H7, H8<br>7,25 - 7,95:m, 4H aromatiques AA'BB' |
| 24 | 122°C | C 68,08<br>H 5,98 | C 67,92<br>H 6,16 | 1,30:3H, t, SO2CH2CH3, 3,15 q, 2H, SO2CH2CH3<br>3,85:s, 3H, OCH3 4,85, s, 2H, H2 en 2<br>6,40 - 8,20:m, 9H, H éthylénique et aromatiques |

s : singulet ; t : triplet ; q : quadruplet ; m : massif

**EXEMPLE 25 : (PHENYL - 1 PROPENYL - 2) - 3 METHOXY - 7 2H - CHROMENE**

**STADE A : METHOXY - 7 ACETYL - 3 2H - CHROMENE**

A température ambiante et sous agitation magnétique, ajouter 1,52 g (10 mmole) d'aldéhyde hydroxy - 2 méthoxy - 4 benzoïque, 0,66 g (10 mmole) d'oxo - 3 butène - 1 et 0,79 g (10 mmole) de pyridine dans une suspension de 0,14 g (1 mmole) de carbonate de potassium dans 100 ml de diméthyl formamide. Porter à reflux en maintenant l'agitation magnétique pendant 6 heures. Verser ensuite le milieu réactionnel dans 200 ml d'une solution aqueuse d'hydroxyde de sodium 0,5 N puis extraire à trois reprises par 100 ml d'éther diéthylique. Réunir les phases organiques, laver à 2 reprises par 100 ml de solution aqueuse saturée de chlorure de sodium et sécher. Evaporer les solvants et purifier par chromatographie (voir tableau A1)

Rendement : 42 %

**STADES B A D :**

Procéder comme dans l'exemple 1 en remplacant l'acétyl - 3 2H - chromène par le méthoxy - 7 acétyl - 3 2H chromène. On obtient le produit attendu.

**EXEMPLES 26 A 28 :**

En procédant comme dans l'exemple 25, en remplaçant au stade D l'aldéhyde benzoïque par :

- l'ester méthylique de l'acide formyl - 4 benzoïque, on obtient la [(méthoxy - 7 2H - chroményl - 3) - 2 propényl] - 4 benzoate de méthyle. **(EXEMPLE 26)**
- l'ester éthylique de l'acide formyl - 4 benzoïque, on obtient la [(méthoxy - 7 2H - chroményl - 3) - 2 propényl] - 4 benzoate d'éthyle. **(EXEMPLE 27)**

l'éthyle formyl - 4 phényl sulfone, on obtient l'éthyl [(méthoxy - 7 2H - chroményl - 3) - 2 propényl] - 4 phényl sulfone. **(EXEMPLE 28)**

Le tableau E visualise les données relatives à la synthèse de ces exemples.

Le tableau F visualise les caractéristiques physico-chimiques de ces produits.

TABLEAU E

SYNTHESE DES EXEMPLES 26 A 28

| EX N° | R | STEREO-CHIMIE | STADE B | STADE C | | STADE D | |
|---|---|---|---|---|---|---|---|
| | | | Rdt (%) | Rdt (%) | F °C | (H) | Rdt (%) |
| 26 | $COOCH_3$ | E | 92 | 95 | 70 | 18 | 48 |
| 27 | $COOC_2H_5$ | E | 92 | 95 | 70 | 45 | 108 |
| 28 | $SO_2C_2H_5$ | E | 92 | 95 | 70 | 04 | 35 |

En procédant comme dans les exemples 1 ou 2, mais en remplaçant au stade D l'aldéhyde benzoïque par :

– l'aldéhyde chloro - 4 benzoïque, on obtient le [(chloro - 4 phényl) - propényl - 2] - 3 2H - chromène de stéréochimie.E + Z **(EXEMPLE 29)** ou **E (EXEMPLE 30)**

– la para tolualdéhyde, on obtient le (p.toluyl - 1 propenyl - 2) - 3 2H - chromène de stéréochimie (E + Z) **(EXEMPLE 31)** ou E **(EXEMPLE 32)**

– la para anisaldéhyde, on obtient le [(méthoxy - 4 phényl) - 1 propényl - 2] - 3 2H - chromène de stéréochimie.E + Z **(EXEMPLE 33)** ou E **(EXEMPLE 34)**

TABLEAU F : CARACTERISTIQUES PHYSICOCHIMIQUES DES EXEMPLES 26 A 28

| Ex N° | F (°C) | ANALYSE ELEMENTAIRE | | SPECTROMETRIE | |
|---|---|---|---|---|---|
| | | CALCULEE | TROUVEE | INFRA-ROUGE ($cm^{-1}$) | RESONANCE MAGNETIQUE NUCLEAIRE *(δ) ppm |
| 26 | 143°C | C 74,98<br>H 5,99 | C 74,88<br>H 6,09 | 1720, 1620, 1120 | 2,10:s, 3H, $CH_3$ éthylénique<br>3,75:s, 3H, $OCH_3$ (7)<br>3,90:s, 3H, $OCH_3$ ester; 5,05:s.e. 2H, $H_2$ (2)<br>6,20 - 8,10:9H éthyléniques et aromatiques |
| 27 | 180°C | C 75,41<br>H 6,33 | C 75,25<br>H 6,10 | | 1,40 t, 3H, $OCH_2$ - $\underline{CH_3}$<br>2,15:m 3H, $CH_3$ éthylène<br>3,85:s, 3H, $OCH_3$ (7)<br>4,45:q, 2H, $O\underline{CH_2}CH_3$<br>5,20:s.e., 2H, H (2);6,50 s.e. 1H éthyline<br>6,60 - 8,35=:massif 8H chromane et benzéniques |
| 28 | 156°C | C 68,15<br>H 5,99 | C 68,21<br>H 6,06 | | 1,30 t, 3H, $SO_2CH_2\underline{CH_3}$ J = $7H_2$<br>2,15:m, 3H, $CH_3$ éthylène<br>3,15 q, 2H, $SO_2$ - $\underline{CH_2}$ J = $7H_2$<br>3,80 s, 3H, $OCH_3$;5,10, s.e. 2H, HZ<br>6,40 - 6,80 m 4H, $H_4$, $H_6$, $H_8$ + M éthylénique<br>6,90 - 8,10 m, 5H, $H_5$ et Har AA'BB' |

s : singulet ; d : doublet ; t : triplet ; q : quadruplet ; m : massif

**EXEMPLE 35 : ACIDE [(2H - CHROMENYL - 3) - 2 PROPENYL] - 4 BENZOIQUE**

Par hydrolyse alcaline en présence de potasse alcoolique du [(2H chroményl - 3) - 2 propényl] - 4 benzoate de méthyle, on obtient le produit du titre.

Rendement : 45 %

Point de fusion : 175°C

Composition centésimale :

Calculée : C : 75,22 H : 5,64

Trouvée : C : 75,72 H : 5,63

Caractéristiques spectrales :

Résonance magnétique nucléaire :

δ = 2,15 ppm 3H singulet, $CH_3$

**ETUDE PHARMACOLOGIQUE :**

**EXEMPLE 36 : INHIBITION DE CROISSANCE DE LA LIGNEE L1210**

La croissance de cette lignée leucémique de souris est appréciée par la capacité des cellules à incorporer la thymidine tritiée. Le taux d'incorporation est mesuré 48 heures après l'introduction des composés à tester dans le milieu de culture.

Le tableau F réunit les valeurs d'inhibition de croissance 50 % ($IC_{50}$) pour chaque produit.

| EX N° | IC50 (µM) 48 Heures |
|---|---|
| 1 | <1 |
| 3 | 20 |
| 6 | 25 |
| 8 | 25 |
| 10 | 18 |
| 13 | 3,8 |
| 16 | 3,8 |
| 17 | 7,5 |
| 18 | 6 |
| 24 | 8 |
| Etrétinate | 80 |

Cette étude montre que les exemples étudiés ont une activité nettement supérieure à celle de l'étrétinate.

**EXEMPLE 37 : ETUDE DE LA TOXICITE SUR LES HUMERI DE FOETUS DE RATS DE 21 JOURS DE GESTATION**

La toxicité des rétinoïdes peut être évaluée sur les humeri de foetus de rats de 21 jours de gestation.

La toxicité des rétinoïdes peut être évaluée sur les huméri de foetus de rats explantés in vitro d'après KISTLER.

L'activité des rétinoïdes se traduit par une libération des protéoglycanes de la matière osseuse. Cette libération est appréciée après 7 jours de culture in vitro par le dosage de la concentration de protéoglycanes dans le milieu, par la méthode de WITHEMAN (1973).

Les composés de la présente invention s'avèrent en général 3 à 8 fois moins toxiques que l'étrétinate. Le

composé de l'exemple 8 a montré une absence totale de toxicité dans ce test.

**EXEMPLE 38: DETECTION D'UNE TOXICITE DE TYPE HYPERVITAMINIQUE A IN VIVO**

Les effets secondaires limitant l'utilisation des agents rétinoïdes en clinique humaine sont l'apparition d'un syndrome d'hypervitaminose A. Celui-ci peut être reproduit expérimentalement chez l'animal.

Le protocole de traitement choisi est une injection quotidienne pendant cinq jours, reproduit à deux reprises espacées de deux jours. Il est identique à celui utilisé par BOLLAG et Coll (1981). Alors que l'acide rétinoïque et l'étrétinate entraînent une perte de poids rapide, l'apparition d'une alopécie et la fragilisation des os, manifestations de leur toxicité, les composés de la présente invention se sont avérés dépourvus de toxicité de type hypervitaminique A : aucun signe pondéral statistiquement significatif, aucune fracture détectable par radiogrpahie, aucun début d'alopécie n'ont été signalés suite au traitement par les composés de l'invention.

**EXEMPLE 39 : EXEMPLE DE COMPOSITION PHARMACEUTIQUE**

Comprimé dosé à 5 mg de (Phényl - 1 propényl - 2) - 3 2H chromène

Formule de préparation pour 1000 comprimés

| | |
|---|---|
| (Phényl - 1 propényl - 2) - 3 2H chromène | 5 g |
| Amidon de blé | 20 g |
| Amidon de maïs | 20 g |
| Lactose | 75 g |
| Stéarate de magnésium | 2 g |
| Silice | 1 g |
| Hydroxy propyl cellulose | 2 g |

## Revendications

**Revendications pour les Etats contractants suivants : AT, BE, CH, DE, FR, GB, IT, LI, LU, NL, SE**

1/ Composés de formule générale (I) :

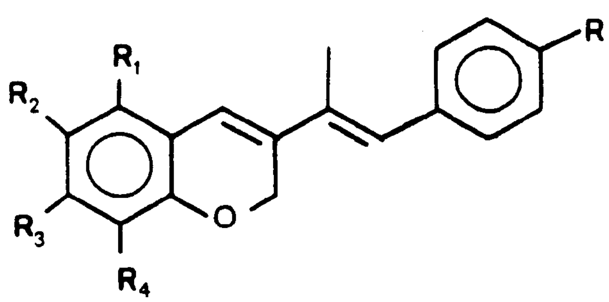

(I)

dans laquelle :

R représente un atome d'hydrogène, un atome d'halogène, ou un groupement hydroxy, alcoyle inférieur, alcoyloxy inférieur, carboxyle, alcoyloxy inférieur carbonyl, arylalcoyloxy inférieur carbonyle, amino-

carbonyle, mono ou dialcoyle inférieur amino carbonyle, aryl alcoyle inférieur amino carbonyle, N hétérocycylaminocarbonyl, thio, alcoyle inférieur thio, sulfonyle, alcoyle inférieur sulfonyl,

$R_1$, $R_2$, $R_3$, $R_4$ identiques ou différents représentent un atome d'hydrogène, un atome d'halogène, un groupement alcoyle inférieur, alkényl inférieur, alcoyloxy inférieur ou alkényloxy inférieur, éventuellement substitués par un ou plusieurs atomes d'halogène,

leurs isomères, énantiomères, diastéréoisomères ainsi que lorsque R représente un carboxyle, leurs sels d'addition à une base pharmaceutiquement acceptable, et lorsque R contient un groupement basique, leurs sels d'addition à un acide pharmaceutiquement acceptable.

2/ Composés selon la revendication 1, dans laquelle R représente un groupement éthoxy carbonyle.

3/ Composés selon la revendication 1, dans laquelle R représente un groupement méthoxy carbonyle.

4/ Composés selon la revendication 1, dans laquelle R représente un groupement alkyle inférieur sulfonyle.

5/ Composés selon la revendication 1, dans laquelle R représente un groupement alkyle inférieur amino carbonyle.

6/ Composés selon la revendication 1, dans laquelle R représente un atome d'hydrogène.

7/ Composés selon la revendication 1, dans laquelle la configuration est E.

8/ Composé selon l'une des revendications 1, 6, 7 qui est le (Phényl - 1 propényl - 2) - 3 2H chromène (E).

9/ Composé selon l'une des revendications 1, 5 et 7 qui est l'[(Ethylaminocarbonyl - 4 phényl) - 1 propényl - 2] - 3 2H chromène (E).

10/ Composé selon l'une des revendications 1, 4 et 7 qui est l'[(éthyl sulfonyl - 4 phényl) - 1 propényl - 2] - 3 2H chromène (E).

11/ Composé selon l'une des revendications 1, 4 et 7 qui est l'[(éthyl sulfonyl - 4 phényl) - 1 propényl - 2] - 3 diméthyl 6,8 2H chromène.

12/ Composé selon l'une des revendications 1, 4 et 7 qui est l'[(éthyl sulfonyl - 4 phényl) - 1 propényl - 2] - 3 chloro - 6 méthyl - 8 2H chromène.

13/ Procédé de préparation de dérivés de formule (I) caractérisé en ce que l'on condense par chauffage à reflux un dérivé de formule (II) :

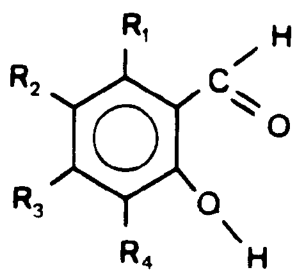

(II)

dans lequel $R_1$, $R_2$, $R_3$ et $R_4$ ont la même signification que dans la formule (I),

avec l'oxo - 3 butène - 1 de formule (III) :

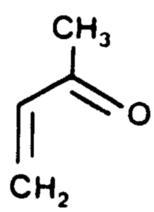

(III)

en présence d'un agent alcalin préférentiellement choisi parmi les carbonates alcalins et alcalino terreux, ou les bases organiques, comme par exemple, la triéthylamine, ou la pyridine, ou un mélange constitué d'un carbonate alcalin ou alcalino terreux et d'une base organique, dans un solvant polaire, préférentiellement choisi parmi la butanone - 2 le diméthylformamide, l'acétone, le diméthylsulfoxide,

pour conduire après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le dichlorométhane, le chloroforme, lavage de la phase organique, évaporation du solvant et purification du résidu par cristallisation, ou distillation, ou chromatographie sur colonne de silice ou d'alumine,

à un dérivé de formule (IV) :

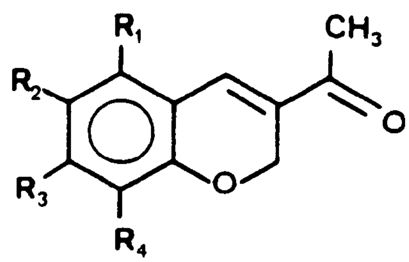

(IV)

dans laquelle $R_1$, $R_2$, $R_3$ et $R_4$ ont la même signification que dans la formule (I),

lequel, après dissolution dans un solvant organique convenable préférentiellement choisi parmi les alcools aliphatiques inférieurs est soumis, préférentiellement en présence d'un agent alcalin tel que l'hydroxyde de sodium, à hydrogénation catalytique préférentiellement par un hydrure mixte de métal alcalin tel que le borohydrure de sodium pour conduire,

après une éventuelle évaporation du milieu réactionnel, extraction par un solvant organique convenable tel que l'éther diéthylique, le chlorure de méthylène, le chloroforme, lavage puis évaporation de la phase organique et purification

à un dérivé de formule (V) :

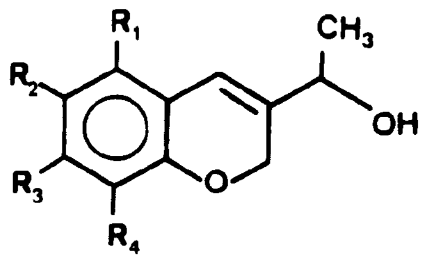

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs, à température ambiante et sous agitation pour conduire, après évaporation du solvant et purification par chromatographie sur gel de silice ou par cristallisation dans un solvant ou un mélange de solvants appropriés,

à un dérivé de formule (VI) :

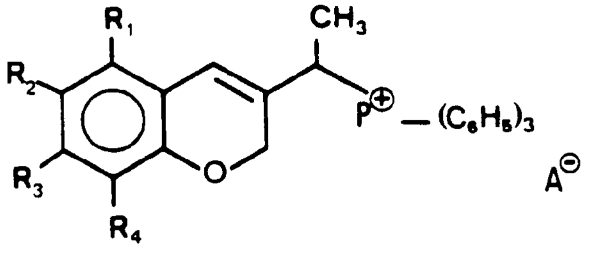

(VI)

dans laquelle $R_1$, $R_2$, $R_3$ et $R_4$ ont la même définition que dans la formule (I), et A- représente l'anion d'un hydracide,

qui, après mise en suspension dans un solvant organique, est traité par une solution de n butyllithium dans l'hexane préférentiellement à température ambiante puis traité par un dérivé de formule (VII) :

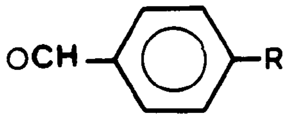

(VII)

dans laquelle R a la même signification que dans la formule (I),

préférentiellement à température ambiante, pour conduire, après dilution dans l'eau ou élimination du solvant, extraction par un solvant organique choisi parmi éther diéthylique, éther diisopropylique, chloroforme, chlorure de méthylène et purification par chromatographie sur colonne de silice à un dérivé de formule (I),

que l'on peut, si on le désire :

– soit, lorsque R représente un groupement carboxylique, salifier par une base pharmaceutiquement acceptable, ou lorsque R comporte un groupement basique, salifier par un acide pharmaceutiquement acceptable,

– soit séparer en ses isomères, diastéréoisomères, énantiomères par une technique de cristallisation ou de chromatographie puis, si on le désire, lorsque R représente un groupement carboxyle, salifier par une base pharmaceutiquement acceptable, ou lorsque R comporte un groupement basique, salifier par un acide pharmaceutiquement acceptable.

14/ Procédé de préparation des dérivés de formule (I/A) :

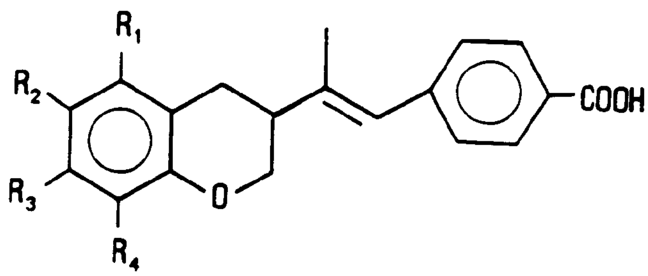

(I/A)

dans lesquels $R_1$, $R_2$, $R_3$ et $R_4$ ont la même définition que pour les composés de formule (I) selon la revendication 1, caractérisé en ce que l'on soumet à hydrolyse alcaline un dérivé de formule (I/B) :

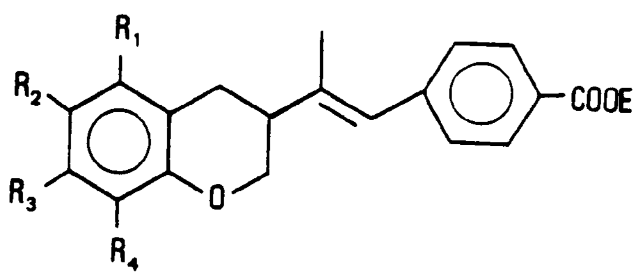

(I/B)

pour lequel E représente un groupement alkyle inférieur et $R_1$, $R_2$, $R_3$, $R_4$ ont la même signification que précédemment,

pour obtenir un dérivé de formule (I/A),

que l'on sépare le cas échéant en ses isomères, énantiomères ou diastéréroisomères,

et que l'on salifie, si on le désire, par une base pharmaceutiquement acceptable.

15/ Composition pharmaceutique contenant comme principe actif au moins un composé selon l'une des revendications 1 à 12 en combinaison avec un ou plusieurs excipients ou véhicules inertes, non-toxiques, pharmaceutiquement acceptables.

16/ Composition pharmaceutique selon la revendication 15 contenant au moins un principe actif selon l'une des revendications 1 à 12 utilisables dans le traitements des tumeurs, des néoplasmes ainsi que dans les troubles cutanés divers.

**Revendications pour l'Etat contractant suivant : GR**

1/ Procédé de préparation de composés de formule générale (I) :

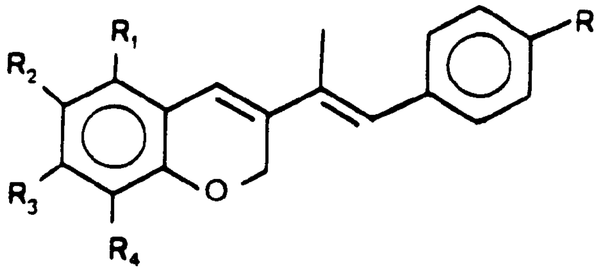

(I)

dans laquelle :

R représente un atome d'hydrogène, un atome d'halogène, ou un groupement hydroxy, alcoyle inférieur, alcoyloxy inférieur, carboxyle, alcoyloxy inférieur carbonyl, arylalcoyloxy inférieur carbonyle, aminocarbonyle, mono ou dialcoyle inférieur amino carbonyle, aryl alcoyle inférieur amino carbonyle, N hétérocycylaminocarbonyl, thio, alcoyle inférieur thio, sulfonyle, alcoyle inférieur sulfonyl,

$R_1$, $R_2$, $R_3$, $R_4$ identiques ou différents représentent un atome d'hydrogène, un atome d'halogène, un groupement alcoyle inférieur, alkényl inférieur, alcoyloxy inférieur ou alkényloxy inférieur, éventuellement substitués par un ou plusieurs atomes d'halogène,

leurs isomères, énantiomères, diastéréoisomères ainsi que lorsque R représente un carboxyle, leurs sels d'addition à une base pharmaceutiquement acceptable, et lorsque R contient un groupement basique, leurs sels d'addition à un acide pharmaceutiquement acceptable,

caractérisé en ce que l'on condense par chauffage à reflux un dérivé de formule (II) :

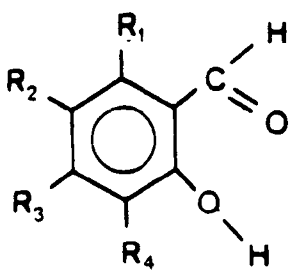

(II)

dans lequel $R_1$, $R_2$, $R_3$ et $R_4$ ont la même signification que dans la formule (I),

avec l'oxo - 3 butène - 1 de formule (III) :

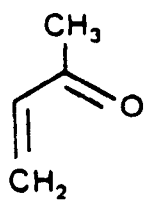

(III)

en présence d'un agent alcalin préférentiellement choisi parmi les carbonates alcalins et alcalino terreux, ou les bases organiques, comme par exemple, la triéthylamine, ou la pyridine, ou un mélange constitué d'un carbonate alcalin ou alcalino terreux et d'une base organique, dans un solvant polaire, préférentiellement choisi parmi la butanone - 2 le diméthylformamide, l'acétone, le diméthylsulfoxide,

pour conduire après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le dichlorométhane, le chloroforme, lavage de la phase organique, évaporation du solvant et purification du résidu par cristallisation, ou distillation, ou chromatographie sur colonne de silice ou d'alumine,

à un dérivé de formule (IV) :

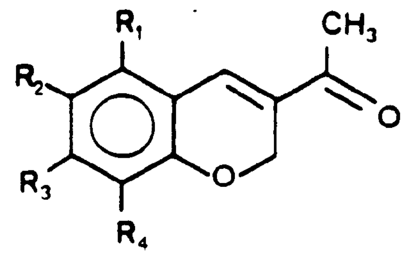

(IV)

dans laquelle $R_1$, $R_2$, $R_3$ et $R_4$ ont la même signification que dans la formule (I),

lequel, après dissolution dans un solvant organique convenable préférentiellement choisi parmi les alcools aliphatiques inférieurs est soumis, préférentiellement en présence d'un agent alcalin tel que l'hydroxyde de sodium, à hydrogénation catalytique préférentiellement par un hydrure mixte de métal alcalin tel que le borohydrure de sodium pour conduire,

après une éventuelle évaporation du milieu réactionnel, extraction par un solvant organique convenable tel que l'éther diéthylique, le chlorure de méthylène, le chloroforme, lavage puis évaporation de la phase organique et purification

à un dérivé de formule (V) :

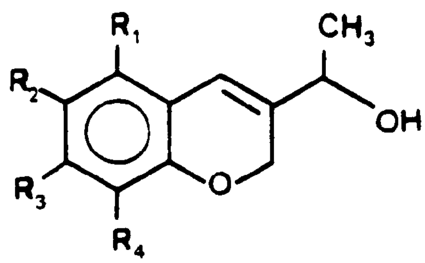

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs, à température ambiante et sous agitation pour conduire, après évaporation du solvant et purification par chromatographie sur gel de silice ou par cristallisation dans un solvant ou un mélange de solvants appropriés,

à un dérivé de formule (VI) :

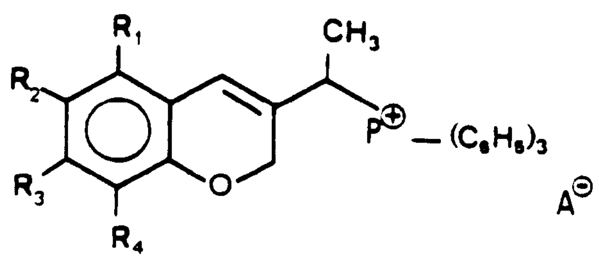

(VI)

dans laquelle $R_1$, $R_2$, $R_3$ et $R_4$ ont la même définition que dans la formule (I), et A- représente l'anion d'un hydracide,

qui, après mise en suspension dans un solvant organique, est traité par une solution de n butyllithium dans l'hexane préférentiellement à température ambiante puis traité par un dérivé de fomule (VII) :

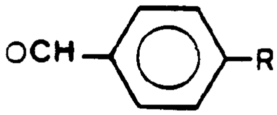

(VII)

dans laquelle R a la même signification que dans la formule (I),

préférentiellement à température ambiante, pour conduire, après dilution dans l'eau ou élimination du solvant, extraction par un solvant organique choisi parmi éther diéthylique, éther diisopropylique, chloroforme, chlorure de méthylène et purification par chromatographie sur colonne de silice à un dérivé de formule (I),

qui, lorsque R représente un groupement COOE dans lequel E représente un groupement alkyle inférieur peut, si on le désire, être soumis à hydrolyse alcaline pour obtenir un dérivé de formule (I) dans lequel R représente un groupement COOH,

dérivé de formule (I) que l'on peut, si on le désire et quelque soit le procédé selon lequel il a été obtenu,

– soit, lorsque R représente un groupement carboxylique, salifier par une base pharmaceutiquement acceptable, ou lorsque R comporte un groupement basique, salifier par un acide pharmaceutiquement acceptable,

– soit séparer en ses isomères, diastéréoisomères, énantiomères par une technique de cristallisation ou de chromatographie puis, si on le désire, lorsque R représente un groupement carboxyle, salifier par une base pharmaceutiquement acceptable, ou lorsque R comporte un groupement basique, salifier par un acide pharmaceutiquement acceptable.

2/ Procédé de préparation selon la revendication 1 des dérivés de formule (I/A) :

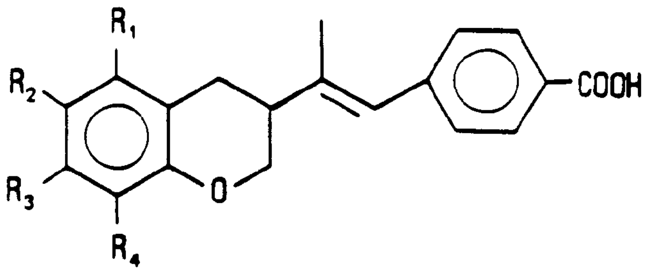

(I/A)

dans lesquels $R_1$, $R_2$, $R_3$ et $R_4$ ont la même définition que pour les composés de formule (I) selon la revendication 1, caractérisé en ce que l'on soumet à hydrolyse alcaline un dérivé de formule (I/B) :

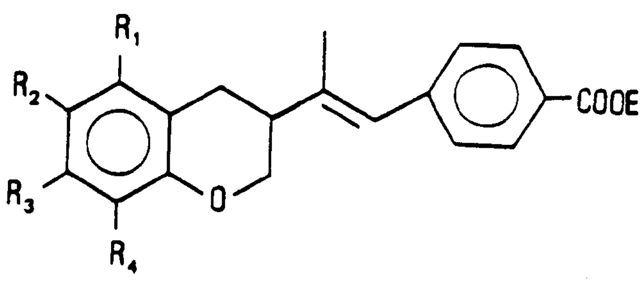

(I/B)

pour lequel E représente un groupement alkyle inférieur et $R_1$, $R_2$, $R_3$, $R_4$ ont la même signification que précédemment,

pour obtenir un dérivé de formule (I/A),

que l'on sépare le cas échéant en ses isomères, énantiomères ou diastéréroisomères,

et que l'on salifie, si on le désire, par une base pharmaceutiquement acceptable.

3/ Procédé selon la revendication 1 de préparation de composés de formule (I), dans laquelle R représente un groupement éthoxy carbonyle.

4/ Procédé selon la revendication 1, de préparation de dérivés de formule (I), dans laquelle R représente un groupement méthoxy carbonyle.

5/ Procédé selon la revendication 1, de préparation de dérivés de formule (I), dans laquelle R représente

un groupement alkyle inférieur sulfonyle.

6/ Procédé selon la revendication 1, de préparation de dérivés de formule (I), dans laquelle R représente un groupement alkyle inférieur amino carbonyle.

7/ Procédé selon la revendication 1, de préparation de dérivés de formule (I), dans laquelle R représente un atome d'hydrogène.

8/ Procédé selon la revendication 1, de préparation de dérivés de formule (I), dans laquelle la configuration est E.

9/ Procédé selon l'une des revendications 1, 7, 8 de préparation du dérivé de formule (I), qui est le (Phényl - 1 propényl - 2) - 3 2H chromène (E).

10/ Procédé selon l'une des revendications 1, 6 et 8 de préparation du dérivé de formule (I), qui est l' [(Ethylaminocarbonyl - 4 phényl) - 1 propényl - 2] - 3 2H chromène (E).

11/ Procédé selon l'une des revendications 1, 5 et 8 de préparation du dérivé de formule (I), qui est l'[(éthyl sulfonyl - 4 phényl) - 1 propényl - 2] - 3 2H chromène (E).

12/ Procédé selon l'une des revendications 1, 5 et 8 de préparation de dérivés de formule (I), qui est l'[(éthyl sulfonyl - 4 phényl) - 1 propényl - 2] - 3 diméthyl 6,8 2H chromène.

13/ Procédé selon l'une des revendications 1, 5 et 8 de préparation de dérivés de formule (I), qui est l'[(éthyl sulfonyl - 4 phényl) - 1 propényl - 2] - 3 chloro - 6 méthyl - 8 2H chromène.

**Revendications pour l'Etat contractant suivant : ES**

1/ Procédé de préparation de composés de formule générale (I) :

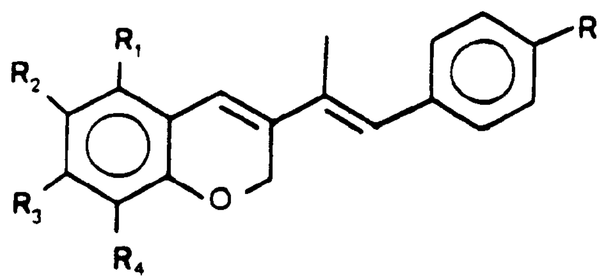

(I)

dans laquelle :

R représente un atome d'hydrogène, un atome d'halogène, ou un groupement hydroxy, alcoyle inférieur, alcoyloxy inférieur, carboxyle, alcoyloxy inférieur carbonyl, arylalcoyloxy inférieur carbonyle, aminocarbonyle, mono ou dialcoyle inférieur amino carbonyle, aryl alcoyle inférieur amino carbonyle, N hétérocycylaminocarbonyl, thio, alcoyle inférieur thio, sulfonyle, alcoyle inférieur sulfonyl,

$R_1$, $R_2$, $R_3$, $R_4$ identiques ou différents représentent un atome d'hydrogène, un atome d'halogène, un groupement alcoyle inférieur, alkényl inférieur, alcoyloxy inférieur ou alkényloxy inférieur, éventuellement substitués par un ou plusieurs atomes d'halogène,

leurs isomères, énantiomères, diastéréoisomères ainsi que lorsque R représente un carboxyle, leurs sels d'addition à une base pharmaceutiquement acceptable, et lorsque R contient un groupement basique, leurs sels d'addition à un acide pharmaceutiquement acceptable,

caractérisé en ce que l'on condense par chauffage à reflux un dérivé de formule (II) :

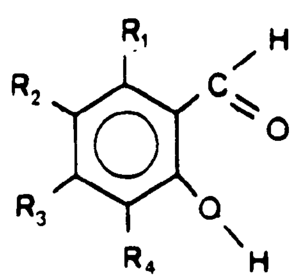

(II)

dans lequel $R_1$, $R_2$, $R_3$ et $R_4$ ont la même signification que dans la formule (I),

avec l'oxo - 3 butène - 1 de formule (III) :

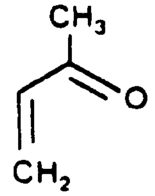

(III)

en présence d'un agent alcalin préférentiellement choisi parmi les carbonates alcalins et alcalino terreux, ou les bases organiques, comme par exemple, la triéthylamine, ou la pyridine, ou un mélange constitué d'un carbonate alcalin ou alcalino terreux et d'une base organique, dans un solvant polaire, préférentiellement choisi parmi la butanone - 2 le diméthylformamide, l'acétone, le diméthylsulfoxide,

pour conduire après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le dichlorométhane, le chloroforme, lavage de la phase organique, évaporation du solvant et purification du résidu par cristallisation, ou distillation, ou chromatographie sur colonne de silice ou d'alumine,

à un dérivé de formule (IV) :

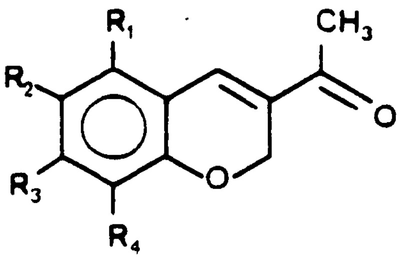

(IV)

dans laquelle $R_1$, $R_2$, $R_3$ et $R_4$ ont la même signification que dans la formule (I),

lequel, après dissolution dans un solvant organique convenable préférentiellement choisi parmi les alcools aliphatiques inférieurs est soumis, préférentiellement en présence d'un agent alcalin tel que l'hydroxyde de sodium, à hydrogénation catalytique préférentiellement par un hydrure mixte de métal alcalin tel que le borohydrure de sodium pour conduire,

après une éventuelle évaporation du milieu réactionnel, extraction par un solvant organique convenable tel que l'éther diéthylique, le chlorure de méthylène, le chloroforme, lavage puis évaporation de la phase organique et purification

à un dérivé de formule (V) :

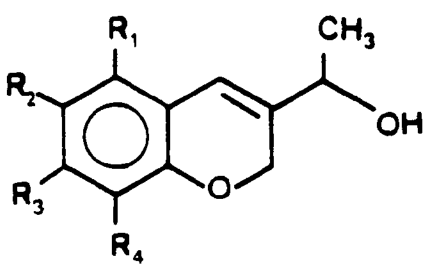

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs, à température ambiante et sous agitation pour conduire, après évaporation du solvant et purification par chromatographie sur gel de silice ou par cristallisation dans un solvant ou un mélange de solvants appropriés,

à un dérivé de formule (VI) :

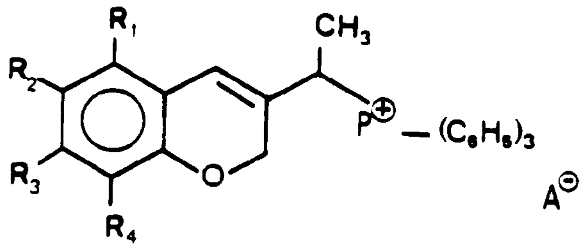

(VI)

dans laquelle $R_1$, $R_2$, $R_3$ et $R_4$ ont la même définition que dans la formule (I), et A- représente l'anion d'un hydracide,

qui, après mise en suspension dans un solvant organique, est traité par une solution de n butyllithium dans l'hexane préférentiellement à température ambiante puis traité par un dérivé de formule (VII) :

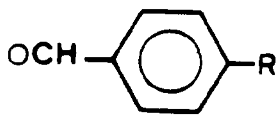

(VII)

dans laquelle R a la même signification que dans la formule (I),

préférentiellement à température ambiante, pour conduire, après dilution dans l'eau ou élimination du solvant, extraction par un solvant organique choisi parmi éther diéthylique, éther diisopropylique, chloroforme, chlorure de méthylène et purification par chromatographie sur colonne de silice à un dérivé de formule (I),

qui, lorsque R représente un groupement COOE dans lequel E représente un groupement alkyle inférieur peut, si on le désire, être soumis à hydrolyse alcaline pour obtenir un dérivé de formule (I) dans lequel R représente un groupement COOH,

dérivé de formule (I) que l'on peut, si on le désire et quelque soit le procédé selon lequel il a été obtenu,

– soit, lorsque R représente un groupement carboxylique, salifier par une base pharmaceutiquement acceptable, ou lorsque R comporte un groupement basique, salifier par un acide pharmaceutiquement acceptable,

– soit séparer en ses isomères, diastéréoisomères, énantiomères par une technique de cristallisation ou de chromatographie puis, si on le désire, lorsque R représente un groupement carboxyle, salifier par une base pharmaceutiquement acceptable, ou lorsque R comporte un groupement basique, salifier par un acide pharmaceutiquement acceptable.

2/ Procédé de préparation selon la revendication 1 des dérivés de formule (I/A) :

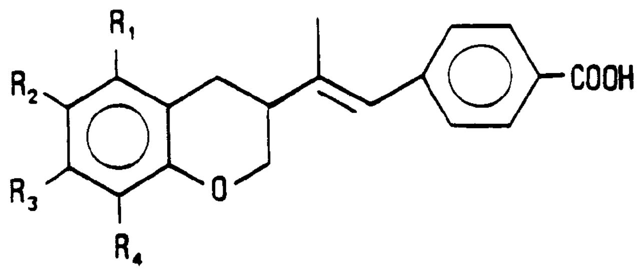

(I/A)

dans lesquels $R_1$, $R_2$, $R_3$ et $R_4$ ont la même définition que pour les composés de formule (I) selon la revendication 1, caractérisé en ce que l'on soumet à hydrolyse alcaline un dérivé de formule (I/B) :

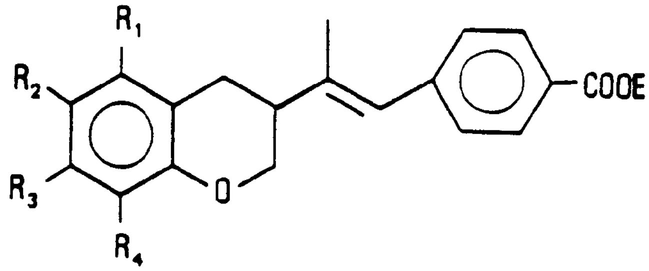

(I/B)

pour lequel E représente un groupement alkyle inférieur et $R_1$, $R_2$, $R_3$, $R_4$ ont la même signification que précédemment,

pour obtenir un dérivé de formule (I/A),

que l'on sépare le cas échéant en ses isomères, énantiomères ou diastéréroisomères,

et que l'on salifie, si on le désire, par une base pharmaceutiquement acceptable.

3/ Procédé selon la revendication 1 de préparation de composés de formule (I), dans laquelle R représente un groupement éthoxy carbonyle.

4/ Procédé selon la revendication 1, de préparation de dérivés de formule (I), dans laquelle R représente un groupement méthoxy carbonyle.

5/ Procédé selon la revendication 1, de préparation de dérivés de formule (I), dans laquelle R représente un groupement alkyle inférieur sulfonyle.

6/ Procédé selon la revendication 1, de préparation de dérivés de formule (I), dans laquelle R représente un groupement alkyle inférieur amino carbonyle.

7/ Procédé selon la revendication 1, de préparation de dérivés de formule (I), dans laquelle R représente un atome d'hydrogène.

8/ Procédé selon la revendication 1, de préparation de dérivés de formule (I), dans laquelle la configuration est E.

9/ Procédé selon l'une des revendications 1, 7, 8 de préparation du dérivé de formule (I), qui est le (Phényl - 1 propényl - 2) - 3 2H chromène (E).

10/ Procédé selon l'une des revendications 1, 6 et 8 de préparation du dérivé de formule (I), qui est l' [(Ethylaminocarbonyl - 4 phényl) - 1 propényl - 2] - 3 2H chromène (E).

11/ Procédé selon l'une des revendications 1, 5 et 8 de préparation du dérivé de formule (I), qui est l'[(éthyl sulfonyl - 4 phényl) - 1 propényl - 2] - 3 2H chromène (E).

12/ Procédé selon l'une des revendications 1, 5 et 8 de préparation de dérivés de formule (I), qui est l'[(éthyl sulfonyl - 4 phényl) - 1 propényl - 2] - 3 diméthyl 6,8 2H chromène.

13/ Procédé selon l'une des revendications 1, 5 et 8 de préparation de dérivés de formule (I), qui est l'[(éthyl sulfonyl - 4 phényl) - 1 propényl - 2] - 3 chloro - 6 méthyl - 8 2H chromène.

## Claims

### Claims for the following Contracting States : AT, BE, CH, DE, FR, GB, IT, LI, LU, NL, SE

**1.** Compounds of the general formula (I):

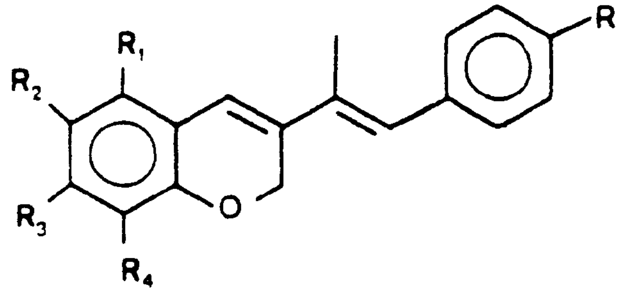

(I)

in which:

R represents a hydrogen atom, a halogen atom, or a hydroxy, lower alkyl, lower alkoxy, carboxy, lower alkoxycarbonyl, aryl-lower alkoxycarbonyl, aminocarbonyl, mono- or di-lower alkylaminocarbonyl, aryl-lower alkylaminocarbonyl, N-heterocyclylaminocarbonyl, thio, lower alkylthio, sulphonyl or lower alkylsulphonyl grouping,

$R_1$, $R_2$, $R_3$ and $R_4$, which are identical or different, represent a hydrogen atom, a halogen atom, a lower alkyl, lower alkenyl, lower alkoxy or lower alkenyloxy grouping, each of which is optionally substituted by one or more halogen atoms,

their isomers, enantiomers, diastereoisomers and also, when R represents a carboxy grouping, their addition salts with a pharmaceutically acceptable base, and, when R contains a basic grouping, their addition salts with a pharmaceutically acceptable acid.

2. Compounds according to claim 1, in which R represents an ethoxycarbonyl grouping.

3. Compounds according to claim 1, in which R represents a methoxycarbonyl grouping.

4. Compounds according to claim 1, in which R represents a lower alkylsulphonyl grouping.

5. Compounds according to claim 1, in which R represents a lower alkylaminocarbonyl grouping.

6. Compounds according to claim 1, in which R represents a hydrogen atom.

7. Compounds according to claim 1, in which the configuration is E.

8. Compound according to one of claims 1, 6 and 7, which is (E)-3-(1-phenyl-2-propenyl)-2H-chromene.

9. Compound according to one of claims 1, 5 and 7, which is (E)-3-[1-(4-ethylaminocarbonylphenyl)-2-propenyl ]-2H-chromene.

10. Compound according to one of claims 1, 4 and 7, which is (E)-3-[1-(4-ethylsulphonylphenyl)-2-propenyl]-2H-chromene.

11. Compound according to one of claims 1, 4 and 7, which is 3-[1-(4-ethylsulphonylphenyl)-2-propenyl]-6,8-dimethyl -2H-chromene.

12. Compound according to one of claims 1, 4 and 7, which is 3-[1-(4-ethylsulphonylphenyl)-2-propenyl]-6-chloro-8-methyl-2H-chromene.

13. Process for the preparation of compounds of the formula (I), characterised in that a compound of the formula (II):

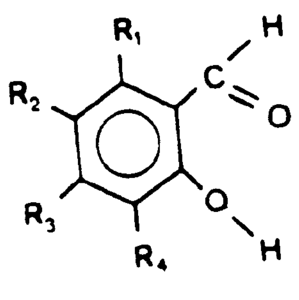

(II)

in which $R_1$, $R_2$, $R_3$ and $R_4$ are as defined in formula (I), is condensed by heating under reflux with 3-oxo-1-butene of the formula (III):

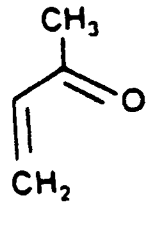

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from 2-butanone, dimethylformamide, acetone and dimethyl sulphoxide,

to yield, after cooling and optionally evaporating the reaction medium, taking up in water to which an alkaline agent, such as sodium hydroxide, has optionally been added, extracting with a suitable organic solvent, such as diethyl ether, dichloromethane or chloroform, washing the organic phase, evaporating the solvent and purifying the residue by crystallisation, or distillation, or chromatography on a silica or aluminium oxide column,

a compound of the formula (IV):

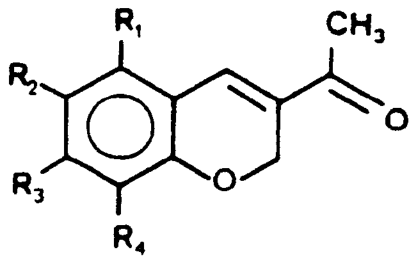

(IV)

in which $R_1$, $R_2$, $R_3$ and $R_4$ are as defined in formula (I),

which, after being dissolved in a suitable organic solvent preferably selected from the lower aliphatic alcohols, is subjected, preferably in the presence of an alkaline agent, such as sodium hydroxide, to catalytic Hydrogenation, preferably using a mixed alkali metal hydride, such as sodium borohydride, to yield,

after optionally evaporating the reaction medium, extracting with a suitable organic solvent, such as diethyl ether, methylene chloride or chloroform, washing and then evaporating the organic phase and purifying,

a compound of the formula (V):

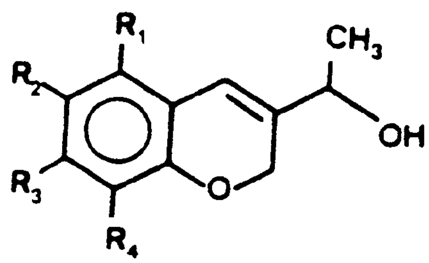

(V)

in which $R_1$, $R_2$, $R_3$ and $R_4$ are as defined above,

which is subjected to the action of a triphenylphosphonium salt in a solvent preferably selected from the lower aliphatic alcohols, at ambient temperature and with stirring, to yield, after evaporating the solvent and purifying by chromatography on silica gel or by crystallisation in a suitable solvent or a mixture of suitable solvents,

a compound of the formula (VI):

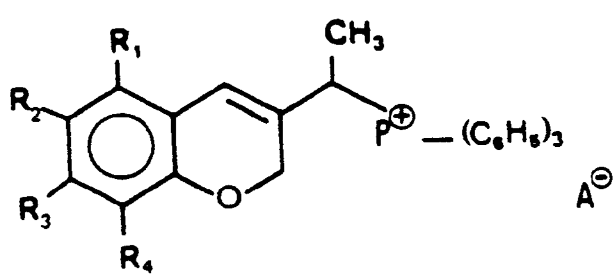

(VI)

in which $R_1$, $R_2$, $R_3$ and $R_4$ are as defined in formula (I), and $A^{\ominus}$ represents the anion of a hydracid,

which, after being suspended in an organic solvent, is treated with a solution of n-butyllithium in hexane, preferably at ambient temperature, and is then treated with a compound of the formula (VII):

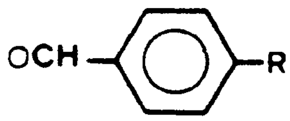

(VII)

in which R is as defined in formula (I),

preferably at ambient temperature, to yield, after diluting in water or eliminating the solvent, extracting with an organic solvent selected from diethyl ether, diisopropyl ether, chloroform and methylene chloride, and purifying by chromatography on a silica column, a compound of the formula (I),

which may, if desired:

- either, when R represents a carboxy grouping, be converted into a salt using a pharmaceutically acceptable base, or, when R comprises a basic grouping, be converted into a salt using a pharmaceutically acceptable acid,
- or be separated into its isomers, diastereoisomers or enantiomers by a crystallisation or chromatographic technique and then, if desired, when R represents a carboxy grouping, be converted into a salt using a pharmaceutically acceptable base, or, when R comprises a basic grouping, be converted into a salt using a pharmaceutically acceptable acid.

**14.** Process for the preparation of the compounds of the formula (I/A):

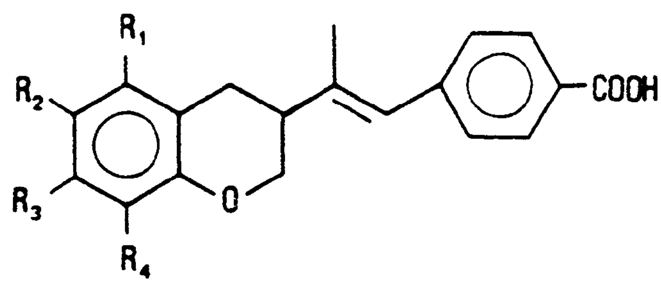

(I/A)

in which $R_1$, $R_2$, $R_3$ and $R_4$ are as defined for the compounds of the formula (I) according to claim 1, characterised in that a compound of the formula (I/B):

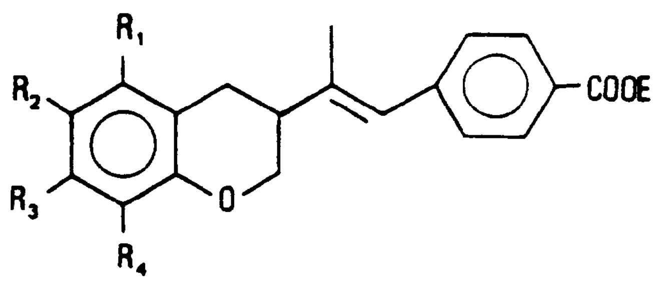

(I/B)

in which E represents a lower alkyl grouping and $R_1$, $R_2$, $R_3$ and $R_4$ are as defined above, is subjected to alkaline hydrolysis,

to obtain a compound of the formula (I/A),

which is separated, where appropriate, into its isomers, enantiomers or diastereoisomers,

and which, if desired, is converted into a salt using a pharmaceutically acceptable base.

**15.** Pharmaceutical composition containing as active ingredient at least one compound according to one of claims 1 to 12 in combination with one or more pharmaceutically acceptable, inert, non-toxic excipients

or carriers.

16. Pharmaceutical composition according to claim 15 containing at least one active ingredient according to one of claims 1 to 12, which can be used in the treatment of tumours, neoplasms and also in various skin disorders.

**Claims for the following Contracting State : GR**

1. Process for the preparation of compounds of the general formula (I):

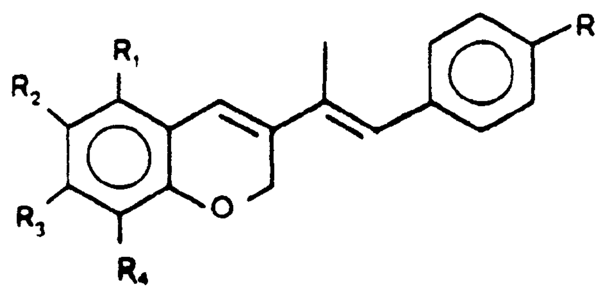

(I)

in which:

R represents a hydrogen atom, a halogen atom, or a hydroxy, lower alkyl, lower alkoxy, carboxy, lower alkoxycarbonyl, aryl-lower alkoxycarbonyl, aminocarbonyl, mono- or di-lower alkylaminocarbonyl, aryl-lower alkylaminocarbonyl, N-heterocyclylaminocarbonyl, thio, lower alkylthio, sulphonyl or lower alkylsulphonyl grouping,

$R_1$, $R_2$, $R_3$ and $R_4$, which are identical or different, represent a hydrogen atom, a halogen atom, a lower alkyl, lower alkenyl, lower alkoxy or lower alkenyloxy grouping, each of which is optionally substituted by one or more halogen atoms,

their isomers, enantiomers, diastereoisomers and also, when R represents a carboxy grouping, their addition salts with a pharmaceutically acceptable base, and, when R contains a basic grouping, their addition salts with a pharmaceutically acceptable acid,

characterised in that a compound of the formula (II):

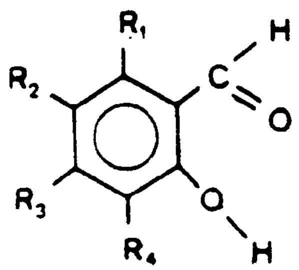

(II)

in which $R_1$, $R_2$, $R_3$ and $R_4$ are as defined in formula (I), is condensed by heating under reflux with 3-oxo-1-butene of the formula (III):

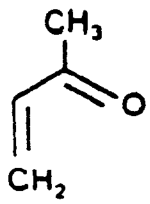

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from 2-butanone, dimethylformamide, acetone and dimethyl sulphoxide,

to yield, after cooling and optionally evaporating the reaction medium, taking up in water to which an alkaline agent, such as sodium hydroxide, has optionally been added, extracting with a suitable organic solvent, such as diethyl ether, dichloromethane or chloroform, washing the organic phase, evaporating the solvent and purifying the residue by crystallisation, or distillation, or chromatography on a silica or aluminium oxide column,

a compound of the formula (IV):

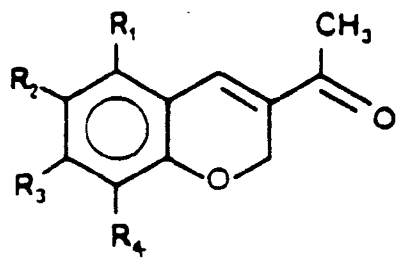

(IV)

in which $R_1$, $R_2$, $R_3$ and $R_4$ are as defined in formula (I),

which, after being dissolved in a suitable organic solvent preferably selected from the lower aliphatic alcohols, is subjected, preferably in the presence of an alkaline agent, such as sodium hydroxide, to catalytic hydrogenation, preferably using a mixed alkali metal hydride, such as sodium borohydride, to yield,

after optionally evaporating the reaction medium, extracting with a suitable organic solvent, such as diethyl ether, methylene chloride or chloroform, washing and then evaporating the organic phase and purifying,

a compound of the formula (V):

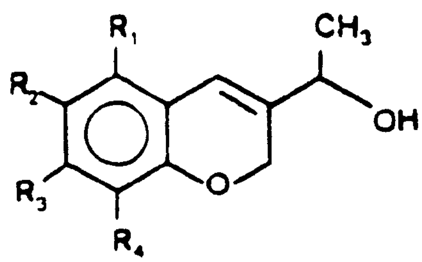

(V)

in which $R_1$, $R_2$, $R_3$ and $R_4$ are as defined above,

which is subjected to the action of a triphenylphosphonium salt in a solvent preferably selected from the lower aliphatic alcohols, at ambient temperature and with stirring, to yield, after evaporating the solvent and purifying by chromatography on silica gel or by crystallisation in a suitable solvent or a mixture of suitable solvents,

a compound of the formula (VI):

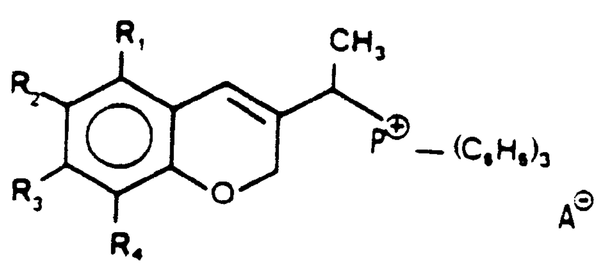

(VI)

in which $R_1$, $R_2$, $R_3$ and $R_4$ are as defined in formula (I), and $A^{\ominus}$ represents the anion of a hydracid,

which, after being suspended in an organic solvent, is treated with a solution of n-butyllithium in hexane, preferably at ambient temperature, and is then treated with a compound of the formula (VII):

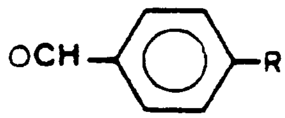

(VII)

in which R is as defined in formula (I),

preferably at ambient temperature, to yield, after diluting in water or eliminating the solvent, extracting with an organic solvent selected from diethyl ether, diisopropyl ether, chloroform and methylene chloride, and purifying by chromatography on a silica column, a compound of the formula (I),

which, when R represents a COOE grouping in which E represents a lower alkyl grouping, may, if desired, be subjected to alkaline hydrolysis to obtain a compound of the formula (I) in which R represents a COOH grouping,

which compound of the formula (I) may, if desired, and regardless of the process by which it has been obtained,

- either, when R represents a carboxy grouping, be converted into a salt using a pharmaceutically acceptable base, or, when R comprises a basic grouping, be converted into a salt using a pharmaceutically acceptable acid,
- or be separated into its isomers, diastereoisomers or enantiomers by a crystallisation or chromatographic technique and then, if desired, when R represents a carboxy grouping, be converted into a salt using a pharmaceutically acceptable base, or, when R comprises a basic grouping, be converted into a salt using a pharmaceutically acceptable acid.

**2.** Process according to claim 1 for the preparation of the compounds of the formula (I/A):

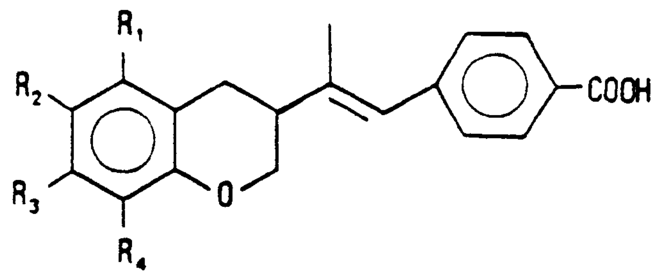

(I/A)

in which $R_1$, $R_2$, $R_3$ and $R_4$ are as defined for the compounds of the formula (I) according to claim 1, characterised in that a compound of the formula (I/B):

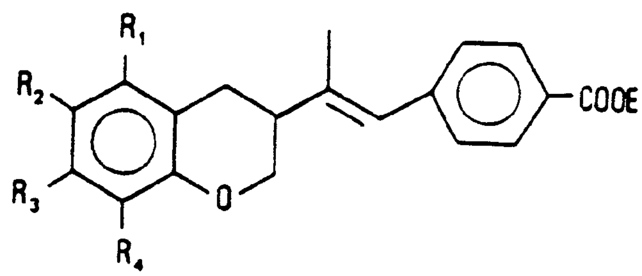

(I/B)

in which E represents a lower alkyl grouping and $R_1$, $R_2$, $R_3$ and $R_4$ are as defined above, is subjected to alkaline hydrolysis,

to obtain a compound of the formula (I/A),

which is separated, where appropriate, into its isomers, enantiomers or diastereoisomers,

and which, if desired, is converted into a salt using a pharmaceutically acceptable base.

**3.** Process according to claim 1 for the preparation of compounds of the formula (I) in which R represents an ethoxycarbonyl grouping.

4. Process according to claim 1 for the preparation of compounds of the formula (I) in which R represents a methoxycarbonyl grouping.

5. Process according to claim 1 for the preparation of compounds of the formula (I) in which R represents a lower alkylsulphonyl grouping.

6. Process according to claim 1 for the preparation of compounds of the formula (I) in which R represents a lower alkylaminocarbonyl grouping.

7. Process according to claim 1 for the preparation of compounds of the formula (I) in which R represents a hydrogen atom.

8. Process according to claim 1 for the preparation of compounds of the formula (I) in which the configuration is E.

9. Process according to one of claims 1, 7 and 8 for the preparation of the compound of the formula (I) which is (E)-3-(1-phenyl-2-propenyl)-2H-chromene.

10. Process according to one of claims 1, 6 and 8 for the preparation of the compound of the formula (I) which is (E)-3-[1-(4-ethylaminocarbonylphenyl)-2-propenyl]-2H-chromene.

11. Process according to one of claims 1, 5 and 8 for the preparation of the compound of the formula (I) which is (E)-3-[1-(4-ethylsulphonylphenyl)-2-propenyl]-2H-chromene.

12. Process according to one of claims 1, 5 and 8 for the preparation of the compound of the formula (I) which is 3-[1-(4-ethylsulphonylphenyl)-2-propenyl]-6,8-dimethyl -2H-chromene.

13. Process according to one of claims 1, 5 and 8 for the preparation of the compound of the formula (I) which is 3-[1-(4-ethylsulphonylphenyl)-2-propenyl]-6-chloro-8-methyl -2H-chromene.

**Claims for the following Contracting State : ES**

1. Process for the preparation of compounds of the general formula (I):

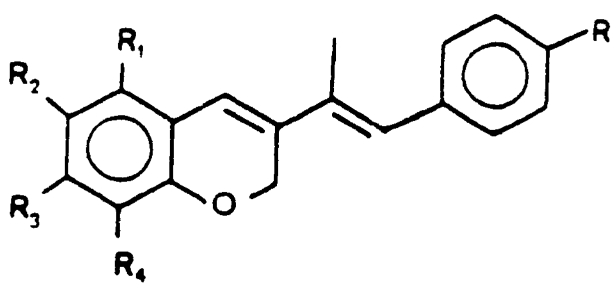

(I)

in which:

R represents a hydrogen atom, a halogen atom, or a hydroxy, lower alkyl, lower alkoxy, carboxy, lower alkoxycarbonyl, aryl-lower alkoxycarbonyl, aminocarbonyl, mono- or di-lower alkylaminocarbonyl, aryl-lower alkylaminocarbonyl, N-heterocyclylaminocarbonyl, thio, lower alkylthio, sulphonyl or lower alkylsulphonyl grouping,

$R_1$, $R_2$, $R_3$ and $R_4$, which are identical or different, represent a hydrogen atom, a halogen atom, a lower alkyl, lower alkenyl, lower alkoxy or lower alkenyloxy grouping, each of which is optionally substituted by one or more halogen atoms,

their isomers, enantiomers, diastereoisomers and also, when R represents a carboxy grouping, their addition salts with a pharmaceutically acceptable base, and, when R contains a basic grouping, their addition salts with a pharmaceutically acceptable acid,

characterised in that a compound of the formula (II):

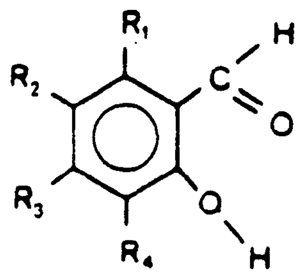

(II)

in which $R_1$, $R_2$, $R_3$ and $R_4$ are as defined in formula (I), is condensed by heating under reflux with 3-oxo-1-butene of the formula (III):

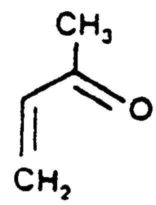

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from 2-butanone, dimethylformamide, acetone and dimethyl sulphoxide,

to yield, after cooling and optionally evaporating the reaction medium, taking up in water to which an alkaline agent, such as sodium hydroxide, has optionally been added, extracting with a suitable organic solvent, such as diethyl ether, dichloromethane or chloroform, washing the organic phase, evaporating the solvent and purifying the residue by crystallisation, or distillation, or chromatography on a silica or aluminium oxide column,

a compound of the formula (IV):

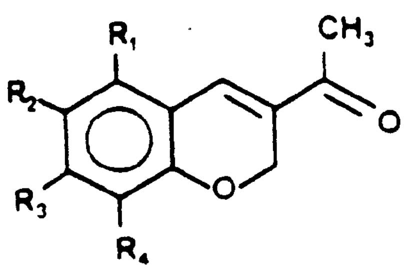

(IV)

in which $R_1$, $R_2$, $R_3$ and $R_4$ are as defined in formula (I),

which, after being dissolved in a suitable organic solvent preferably selected from the lower aliphatic alcohols, is subjected, preferably in the presence of an alkaline agent, such as sodium hydroxide, to catalytic hydrogenation, preferably using a mixed alkali metal hydride, such as sodium borohydride, to yield,

after optionally evaporating the reaction medium, extracting with a suitable organic solvent, such as diethyl ether, methylene chloride or chloroform, washing and then evaporating the organic phase and purifying,

a compound of the formula (V):

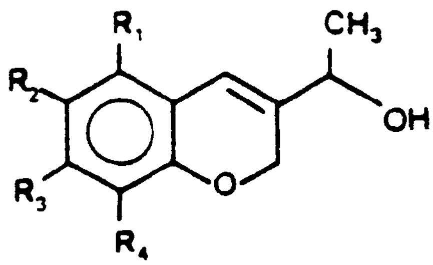

(V)

in which $R_1$, $R_2$, $R_3$ and $R_4$ are as defined above,

which is subjected to the action of a triphenylphosphonium salt in a solvent preferably selected from the lower aliphatic alcohols, at ambient temperature and with stirring, to yield, after evaporating the solvent and purifying by chromatography on silica gel or by crystallisation in a suitable solvent or a mixture of suitable solvents,

a compound of the formula (VI):

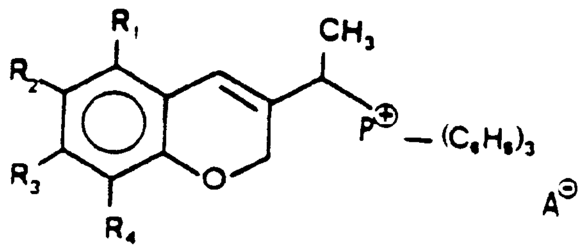

(VI)

in which $R_1$, $R_2$, $R_3$ and $R_4$ are as defined in formula (I), and $A^{\ominus}$ represents the anion of a hydracid,

which, after being suspended in an organic solvent, is treated with a solution of n-butyllithium in hexane, preferably at ambient temperature, and is then treated with a compound of the formula (VII):

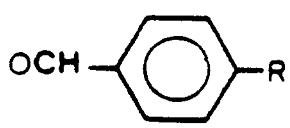

(VII)

in which R is as defined in formula (I),

preferably at ambient temperature, to yield, after diluting in water or eliminating the solvent, extracting with an organic solvent selected from diethyl ether, diisopropyl ether, chloroform and methylene chloride, and purifying by chromatography on a silica column, a compound of the formula (I),

which, when R represents a COOE grouping in which E represents a lower alkyl grouping, may, if desired, be subjected to alkaline hydrolysis to obtain a compound of the formula (I) in which R represents a COOH grouping,

which compound of the formula (I) may, if desired, and regardless of the process by which it has been obtained,

- either, when R represents a carboxy grouping, be converted into a salt using a pharmaceutically acceptable base, or, when R comprises a basic grouping, be converted into a salt using a pharmaceutically acceptable acid,
- or be separated into its isomers, diastereoisomers or enantiomers by a crystallisation or chromatographic technique and then, if desired, when R represents a carboxy grouping, be converted into a salt using a pharmaceutically acceptable base, or, when R comprises a basic grouping, be converted into a salt using a pharmaceutically acceptable acid.

**2.** Process according to claim 1 for the preparation of the compounds of the formula (I/A):

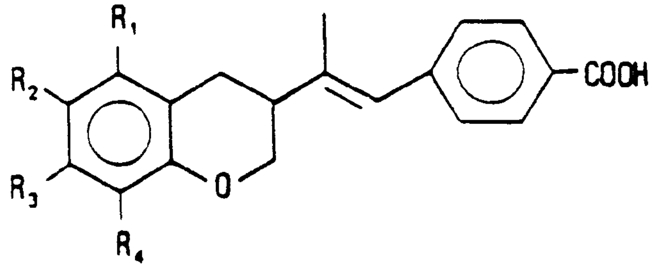

(I/A)

in which $R_1$, $R_2$, $R_3$ and $R_4$ are as defined for the compounds of the formula (I) according to claim 1, characterised in that a compound of the formula (I/B):

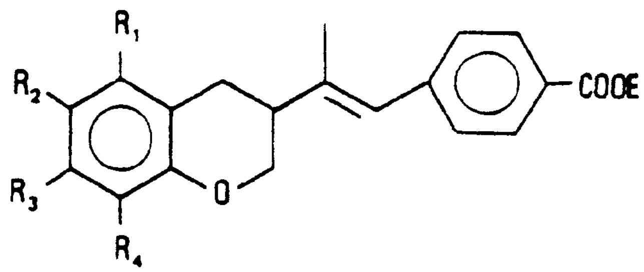

(I/B)

in which E represents a lower alkyl grouping and $R_1$, $R_2$, $R_3$ and $R_4$ are as defined above, is subjected to alkaline hydrolysis,

to obtain a compound of the formula (I/A),
which is separated, where appropriate, into its isomers, enantiomers or diastereoisomers,
and which, if desired, is converted into a salt using a pharmaceutically acceptable base.

3. Process according to claim 1 for the preparation of compounds of the formula (I) in which R represents an ethoxycarbonyl grouping.

4. Process according to claim 1 for the preparation of compounds of the formula (I) in which R represents a methoxycarbonyl grouping.

5. Process according to claim 1 for the preparation of compounds of the formula (I) in which R represents a lower alkylsulphonyl grouping.

6. Process according to claim 1 for the preparation of compounds of the formula (I) in which R represents a lower alkylaminocarbonyl grouping.

7. Process according to claim 1 for the preparation of compounds of the formula (I) in which R represents a hydrogen atom.

8. Process according to claim 1 for the preparation of compounds of the formula (I) in which the configuration is E.

9. Process according to one of claims 1, 7 and 8 for the preparation of the compound of the formula (I) which is (E)-3-(1-phenyl-2-propenyl)-2H-chromene.

10. Process according to one of claims 1, 6 and 8 for the preparation of the compound of the formula (I) which is (E)-3-[1-(4-ethylaminocarbonylphenyl)-2-propenyl]-2H-chromene.

11. Process according to one of claims 1, 5 and 8 for the preparation of the compound of the formula (I) which is (E)-3-[1-(4-ethylsulphonylphenyl)-2-propenyl]-2H-chromene.

12. Process according to one of claims 1, 5 and 8 for the preparation of the compound of the formula (I) which is 3-[1-(4-ethylsulphonylphenyl)-2-propenyl]-6,8-dimethyl -2H-chromene.

13. Process according to one of claims 1, 5 and 8 for the preparation of the compound of the formula (I) which is 3-[1-(4-ethylsulphonylphenyl)-2-propenyl]-6-chloro-8-methyl -2H-chromene.

## Patentansprüche

### Patentansprüche für folgende Vertragsstaaten : AT, BE, CH, DE, FR, GB, IT, LI, LU, NL, SE

1. Verbindungen der allgemeinen Formel (I)

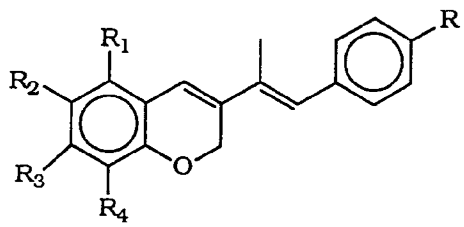

(I)

in der

R ein Wasserstoffatom, ein Halogenatom oder eine Hydroxygruppe, eine Niedrigalkylgruppe, eine Niedrigalkyloxygruppe, eine Carboxylgruppe, eine Niedrigalkyloxycarbonylgruppe, eine Niedrigarylalkyloxycarbonylgruppe, eine Aminocarbonylgruppe, eine Niedrigmono- oder -dialkyl-aminocarbonylgruppe, eine Arylniedrigalkylaminocarbonylgruppe, eine N-Heterocyclylaminocarbonylgruppe, eine Thiogruppe, eine Niedrigalkylthiogruppe, eine Sulfonylgruppe oder eine Niedrigalkylsulfonylgruppe,

$R_I$, $R_2$, $R_3$, $R_4$ die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sind, bedeuten,

deren Isomeren, Enantiomeren und Diastereoisomeren, sowie dann, wenn R eine Carboxylgruppe darstellt, deren Additionssalze mit einer pharmazeutisch annehmbaren Base und dann, wenn R eine basische Gruppe enthält, deren Additionssalze mit einer pharmazeutisch annehmbaren Säure.

2. Verbindungen nach Anspruch I, worin R eine Ethoxycarbonylgruppe bedeutet.

3. Verbindungen nach Anspruch I, worin R eine Methoxycarbonylgruppe bedeutet.

4. Verbindungen nach Anspruch I, worin R eine Niedrigalkylsulfonylgruppe bedeutet.

5. Verbindungen nach Anspruch I, worin R eine Niedrigalkylaminocarbonylgruppe bedeutet.

6. Verbindungen nach Anspruch I, worin R ein Wasserstoffatom bedeutet.

7. Verbindungen nach Anspruch I, die in der Konfiguration E vorliegen.

8. Verbindung nach einem der Ansprüche I, 6 oder 7, nämlich 3-(I-Phenyl -2-propenyl)-2H-chromen (E).

9. Verbindung nach einem der Ansprüche I, 5 und 7, nämlich 3-[I-(4-Ethylaminocarbonyl-phenyl)-2-propenyl]-2H-chromen (E).

10. Verbindung nach einem der Ansprüche I, 4 und 7, nämlich 3-[I-(4-Ethylsulfonyl-phenyl)-2 -propenyl]-2H-chromen (E).

11. Verbindung nach einem der Ansprüche I, 4 und 7, nämlich 3-[I-(4-Ethylsulfonyl-phenyl)-2-propenyl)-6,8-dimethyl-2H-chromen.

12. Verbindung nach einem der Ansprüche I, 4 und 7, nämlich 3-[I-(4-Ethylsulfonyl -phenyl)-2-propenyl]-6-chlor-8-methyl-2H-chromen.

13. Verfahren zur Herstellung der Derivate der Formel (I), **dadurch gekennzeichnet**, daß man ein Derivat der Formel (II)

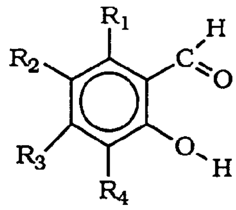

(II)

worin $R_1$, $R_2$, $R_3$ und $R_4$ die bezüglich der allgemeinen Formel (I) angegebenen Bedeutungen besitzen, mit 3-Oxo-buten-l der Formel (III)

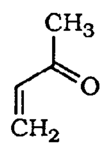

(III)

in Gegenwart eines alkalischen Mittels, welches vorzugsweise aus Alkaliund Erdalkalicarbonaten oder organischen Basen, wie beispielsweise Triethylamin oder Pyridin, oder einer Mischung aus einem Alkali- oder Erdalkalicarbonat und einer organischen Base, ausgewählt ist, in einem polaren Lösungsmittel, welches vorzugsweise aus Butan-2-on, Dimethylformamid, Aceton und Dimethylsulfoxid ausgewählt ist, durch Erhitzen zum Sieden am Rückfluß kondensiert,

so daß man nach Abkühlen und gegebenenfalls Verdampfen des Reaktionsmediums, Wiederaufnahme mit Wasser, welches gegebenenfalls mit einem alkalischen Mittel, wie Natriumhydroxid, versetzt worden ist, Extrahieren mit einem geeigneten organischen Lösungsmittel, wie Diethylether, Dichlormethan oder Chloroform, Waschen der organischen Phase, Verdampfen des Lösungsmittels und Reinigen des Rückstands durch Kristallisation oder Destillation oder Chromatographie über eine mit Siliciumoxid oder Aluminiumoxid gefüllte Säure, ein Derivat der Formel (IV)

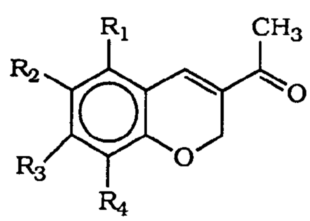

(IV)

in der $R_1$, $R_2$, $R_3$ und $R_4$ die bezüglich der Formel (I) angegebenen Bedeutungen besitzen, erhält,

welches nach dem Auflösen in einem geeigneten organischen Lösungsmittel, das vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung, vorzugsweise mit einem gemischten Alkalimetallhydrid, wie Natriumborhydrid, unterworfen wird, wonach man gegebenenfalls das Reaktionsmedium verdampft, mit einem geeigneten organischen Lösungsmittel, wie Diethylether, Methylenchlorid oder Chloroform, extrahiert, wäscht, die organische Phase verdampft und reinigt unter Bildung eines Derivats der Formel (V)

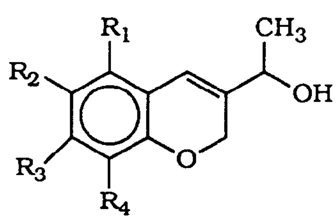

(V)

in der $R_1$, $R_2$, $R_3$ und $R_4$ die oben angegebenen Bedeutungen besitzen,

welches der Einwirkung eines Triphenylphosphoniumsalzes in einem Lösungsmittel, das vorzugsweise aus niedrigmolekularen aliphatischen Alkoholen ausgewählt ist, bei Raumtemperatur und unter Rühren unterworfen wird, so daß man nach dem Verdampfen des Lösungsmittels und Reinigung durch Chromatographie über Kieselgel oder durch Kristallisation aus einem geeigneten Lösungsmittel oder einer geeigneten Lösungsmittelmischung,

ein Derivat der Formel (VI)

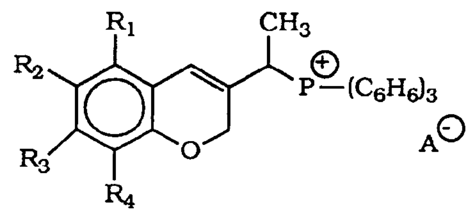

(VI)

erhält, in der $R_l$, $R_2$, $R_3$ und $R_4$ die bezüglich der Formel (I) angegebenen Bedeutungen besitzen und A das Anion einer Wasserstoffsäure bedeutet,

welches man nach der Suspension in einem organischen Lösungsmittel mit einer Lösung von n-Butyllithium in Hexan, vorzugsweise bei Raumtemperatur behandelt und dann mit einem Derivat der Formel (VII)

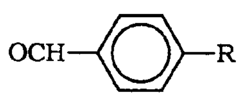

(VII)

in der R die bezüglich der Formel (I) angegebenen Bedeutungen besitzt,

vorzugsweise bei Raumtemperatur behandelt, so daß man nach dem Verdünnen mit Wasser oder durch Beseitigung des Lösungsmittels, Extraktion mit einem aus Diethylether, Diisopropylether, Chloroform und Methylenchlorid ausgewählten organischen Lösungsmittel und Reinigung durch Chromatographie über eine mit Siliciumoxid gefüllte Säule ein Derivat der Formel (I) erhält,

welches man gewünschtenfalls

- entweder, wenn R eine Carboxylgruppe darstellt, mit einer pharmazeutisch annehmbaren Base in ein Salz überführen, oder, wenn R eine basische Gruppe aufweist, mit einer pharmazeutisch annehmbaren Säure in ein Salz umwandeln kann,
- oder mit Methoden der Kristallisation oder der Chromatographie in seine Isomeren, Diastereoisomeren oder Enantiomeren auftrennen und gewünschtenfalls, wenn R eine Carboxylgruppe bedeutet, mit einer pharmazeutisch annehmbaren Base in ein Salz überführen oder, wenn R eine basische Gruppe aufweist, mit einer pharmazeutisch annehmbaren Säure in ein Salz umwandeln kann.

**14.** Verfahren zur Herstellung der Derivate der Formel (I/A):

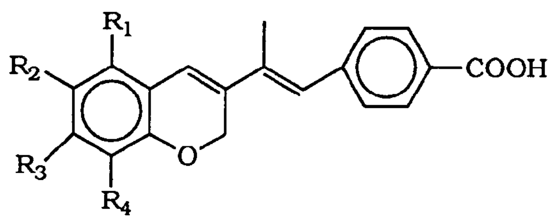

(I/A)

in der $R_l$, $R_2$, $R_3$ und $R_4$ die in Anspruch l bezüglich der Verbindungen der Formel (I) angegebenen Bedeutungen besitzen, **dadurch gekennzeichnet**, daß man ein Derivat der Formel (I/B)

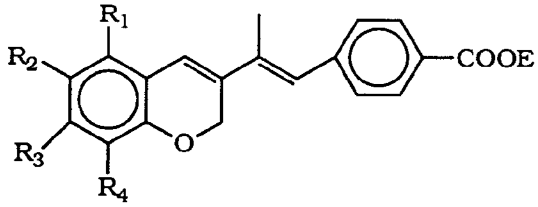

(I/B)

in der E eine Niedrigalkylgruppe darstellt und $R_I$, $R_2$, $R_3$ und $R_4$ die oben angegebenen Bedeutungen besitzen, einer alkalischen Hydrolyse unterwirft zur Bildung eines Derivats der Formel (I/A),

welches man gewünschtenfalls in seine Isomeren, Enantiomeren oder Diastereoisomeren auftrennen kann und

welches man gewünschtenfalls mit einer pharmazeutisch annehmbaren Base in ein Salz überführen kann.

**15.** Pharmazeutische Zubereitung enthaltend als Wirkstoff mindestens eine Verbindung nach einem der Ansprüche I bis I2 in Kombination mit einem oder mehreren inerten, nichttoxischen, pharmazeutisch annehmbaren Trägermaterialien oder Bindemitteln.

**16.** Pharmazeutische Zubereitung nach Anspruch I5 enthaltend mindestens einen Wirkstoff gemäß einem der Ansprüche I bis I2, die zur Behandlung von Tumoren, Neoplasmen sowie verschiedenen Hauterkrankungen geeignet sind.

**Patentansprüche für folgenden Vertragsstaat : GR**

**1.** Verfahren zur Herstellung der Verbindungen der allgemeinen Formel (I)

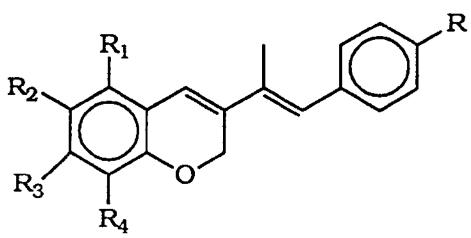

(I)

in der

R ein Wasserstoffatom, ein Halogenatom oder eine Hydroxygruppe, eine Niedrigalkylgruppe, eine Niedrigalkyloxygruppe, eine Carboxylgruppe, eine Niedrigalkyloxycarbonylgruppe, eine Niedrigarylalkyloxycarbonylgruppe, eine Aminocarbonylgruppe, eine Niedrigmono- oder -dialkyl-aminocarbonylgruppe, eine Arylniedrigalkylaminocarbonylgruppe, eine N-Heterocyclylaminocarbonylgruppe, eine Thiogruppe, eine Niedrigalkylthiogruppe, eine Sulfonylgruppe oder eine Niedrigalkylsulfonylgruppe,

$R_I$, $R_2$, $R_3$, $R_4$ die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sind, bedeuten, sowie

deren Isomeren, Enantiomeren und Diastereoisomeren, sowie dann, wenn R eine Carboxylgruppe darstellt, deren Additionssalze mit einer pharmazeutisch annehmbaren Base und dann, wenn R eine basische Gruppe enthält, deren Additionssalze mit einer pharmazeutisch annehmbaren Säure,

**dadurch gekennzeichnet**, daß man ein Derivat der Formel (II)

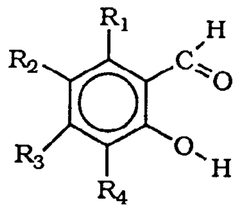

(II)

worin $R_l$, $R_2$, $R_3$ und $R_4$ die bezüglich der allgemeinen Formel (I) angegebenen Bedeutungen besitzen, mit 3-Oxo-buten-l der Formel (III)

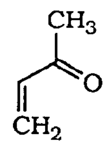

(III)

in Gegenwart eines alkalischen Mittels, welches vorzugsweise aus Alkaliund Erdalkali-Carbonaten oder organischen Basen, wie beispielsweise Triethylamin oder Pyridin, oder einer Mischung aus einem Alkalioder Erdalkali-Carbonat und einer organischen Base, ausgewählt ist, in einem polaren Lösungsmittel, welches vorzugsweise aus Butan-2-oron, Dimethylformamid, Aceton und Dimethylsulfoxid ausgewählt ist, durch Erhitzen zum Sieden am Rückfluß kondensiert,

so daß man nach Abkühlen und gegebenenfalls Verdampfen des Reaktionsmediums, Wiederaufnahme mit Wasser, welches gegebenenfalls mit einem alkalischen Mittel, wie Natriumhydroxid, versetzt worden ist, Extrahieren mit einem geeigneten organischen Lösungsmittel, wie Diethylether, Dichlormethan oder Chloroform, Waschen der organischen Phase, Verdampfen des Lösungsmittels und Reinigen des Rückstands durch Kristallisation oder Destillation oder Chromatographie über eine mit Siliciumoxid oder Aluminiumoxid gefüllte Säure, ein Derivat der Formel (IV)

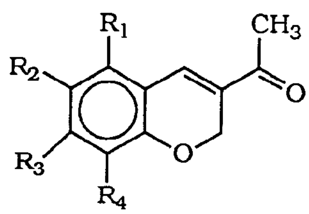

(IV)

in der $R_l$, $R_2$, $R_3$ und $R_4$ die bezüglich der Formel (I) angegebenen Bedeutungen besitzen, erhält,

welches nach dem Auflösen in einem geeigneten organischen Lösungsmittel, das vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung, vorzugsweise mit einem gemischten Alkalimetallhydrid, wie Natriumborhydrid, unterworfen wird, wonach man gegebenenfalls das Reaktionsmedium verdampft, mit einem geeigneten organischen Lösungsmittel, wie Diethylether, Methylenchlorid oder Chloroform, extrahiert, wäscht, die organische Phase vertungen besitzen, dampft und reinigt unter Bildung eines Derivats der Formel (V)

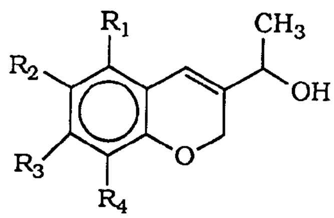

(V)

in der $R_l$, $R_2$, $R_3$ und $R_4$ die oben angegebenen Bedeutungen besitzen,

welches der Einwirkung eines Triphenylphosphoniumsalzes in einem Lösungsmittel das vorzugsweise aus niedrigmolekularen aliphatischen Alkoholen ausgewählt ist, bei Raumtemperatur und unter Rühren unterworfen wird, so daß man nach dem Verdampfen des Lösungsmittels und Reinigung durch Chromatographie über Kieselgel oder durch Kristallisation aus einem geeigneten Lösungsmittel oder einer geeigneten Lösungsmittelmischung,

ein Derivat der Formel (VI)

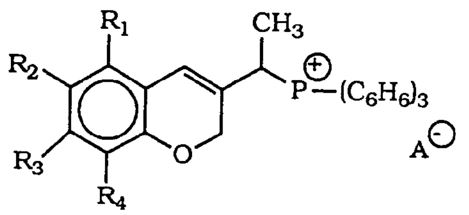

(VI)

erhält, in der $R_l$, $R_2$, $R_3$ und $R_4$ die bezüglich der Formel (I) angegebenen Bedeutungen besitzen und A das Anion einer Wasserstoffsäure bedeutet,

welches man nach der Suspension in einem organischen Lösungsmittel mit einer Lösung von n-Butyllithium in Hexan, vorzugsweise bei Raumtemperatur behandelt und dann mit einem Derivat der Formel (VII)

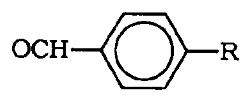

(VII)

in der R die bezüglich der Formel (I) angegebenen Bedeutungen besitzt,

vorzugsweise bei Raumtemperatur behandelt, so daß man nach dem Verdünnen mit Wasser oder durch Beseitigung des Lösungsmittels, Extraktion mit einem aus Diethylether, Diisopropylether, Chloroform und Methylenchlorid ausgewählten organischen Lösungsmittel und Reinigung durch Chromatographie über eine mit Siliciumoxid gefüllte Säule ein Derivat der Formel (I) erhält,

welches man gewünschtenfalls, wenn R eine Gruppe COOE, worin E für eine Niedrigalkylgruppe steht, einer alkalischen Hydrolyse unterziehen kann zur Bildung eines Derivats der Formel (I), worin R eine Gruppe COOH bedeutet,

welches man gewünschtenfalls

- entweder, wenn R eine Carboxylgruppe darstellt, mit einer pharmazeutisch annehmbaren Base in ein Salz überführen, oder, wenn R eine basische Gruppe aufweist, mit einer pharmazeutisch annehmbaren Säure in ein Salz umwandeln kann,
- oder mit Methoden der Kristallisation oder der Chromatographie in seine Isomeren, Diastereoisomeren oder Enantiomeren auftrennen und gewünschtenfalls, wenn R eine Carboxylgruppe bedeutet, mit einer pharmazeutisch annehmbaren Base in ein Salz überführen oder, wenn R eine basische Gruppe aufweist, mit einer pharmazeutisch annehmbaren Säure in ein Salz umwandeln kann.

**2.** Verfahren nach Anspruch I zur Herstellung der Derivate der Formel (I/A):

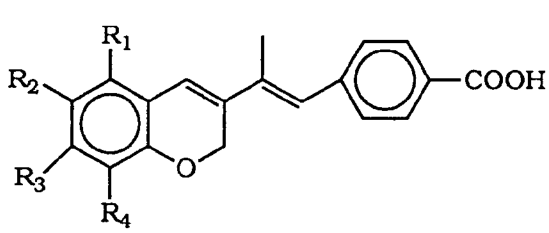

(I/A)

in der $R_l$, $R_2$, $R_3$ und $R_4$ die in Anspruch I bezüglich der Verbindungen der Formel (I) angegebenen Bedeutungen besitzen, **dadurch gekennzeichnet**, daß man ein Derivat der Formel (I/B)

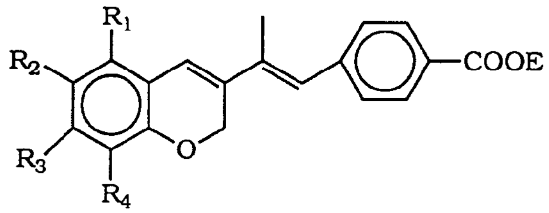

(I/B)

in der E eine Niedrigalkylgruppe darstellt und $R_I$, $R_2$, $R_3$ und $R_4$ die oben angegebenen Bedeutungen besitzen, einer alkalischen Hydrolyse unterwirft zur Bildung eines Derivats der Formel (I/A),

welches man gewünschtenfalls in seine Isomeren, Enantiomeren oder Diastereoisomeren auftrennen kann und

welches man gewünschtenfalls mit einer pharmazeutisch annehmbaren Base in ein Salz überführen kann.

3. Verfahren nach Anspruch I, zur Herstellung von Verbindungen der Formel (I), in der R eine Ethoxycarbonylgruppe bedeutet.

4. Verfahren nach Anspruch I, zur Herstellung von Derivaten der Formel (I), in der R eine Methoxycarbonylgruppe bedeutet.

5. Verfahren nach Anspruch I, zur Herstellung von Derivaten der Formel (I), in der R eine Niedrigalkylsulfonylgruppe bedeutet.

6. Verfahren nach Anspruch I, zur Herstellung von Derivaten der Formel (I), in der R eine Niedrigalkylaminocarbonylgruppe bedeutet.

7. Verfahren nach Anspruch I, zur Herstellung von Derivaten der Formel (I), in der R ein Wasserstoffatom bedeutet.

8. Verfahren nach Anspruch I, zur Herstellung von Derivaten der Formel (I), welche in der Konfiguration (E) vorliegen.

9. Verfahren nach einem der Ansprüche I, 7 und 8 zur Herstellung des Derivats der Formel (I), nämlich 3-(I-Phenyl-2-propenyl)-2H-chromen (E).

10. Verfahren nach einem der Ansprüche I, 6 und 8 zur Herstellung des Derivats der Formel (I), nämlich 3-[I-(4-Ethylaminocarbonyl-phenyl)-2-propenyl]-2H-chromen (E).

11. Verfahren nach einem der Ansprüche I, 5 und 8 zur Herstellung des Derivats der Formel (I), nämlich 3-[I-(4-Ethylsulfonyl-phenyl)-2-propenyl]-2H-chromen (E).

12. Verfahren nach einem der Ansprüche I, 5 und 8 zur Herstellung des Derivats der Formel (I), nämlich 3-[I-(4-Ethylsulfonyl-phenyl)-2-propenyl]-6,8-dimethyl-2H-chromen.

13. Verfahren nach einem der Ansprüche I, 5 und 8 zur Herstellung des Derivats der Formel (I), nämlich 3-[I-(4-Ethylsulfonyl-phenyl)-2-propenyl]-6-chlor-8-methyl-2H-chromen.

**Patentansprüche für folgenden Vertragsstaat : ES**

1. Verfahren zur Herstellung der Verbindungen der allgemeinen Formel (I)

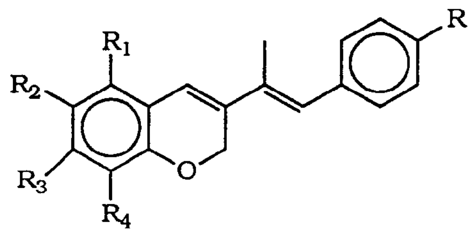

(I)

in der

R ein Wasserstoffatom, ein Halogenatom oder eine Hydroxygruppe, eine Niedrigalkylgruppe, eine Niedrigalkyloxygruppe, eine Carboxylgruppe, eine Niedrigalkyloxycarbonylgruppe, eine Niedrigarylalkyloxycarbonylgruppe, eine Aminocarbonylgruppe, eine Niedrigmono- oder -dialkyl-aminocarbonylgruppe, eine Arylniedrigalkylaminocarbonylgruppe, eine N-Heterocyclylaminocarbonylgruppe, eine Thiogruppe, eine Niedrigalkylthiogruppe, eine Sulfonylgruppe oder eine Niedrigalkylsulfonylgruppe,

$R_I$, $R_2$, $R_3$, $R_4$ die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sind, bedeuten, sowie

deren Isomeren, Enantiomeren und Diastereoisomeren, sowie dann, wenn R eine Carboxylgruppe darstellt, deren Additionssalze mit einer pharmazeutisch annehmbaren Base und dann, wenn R eine basische Gruppe enthält, deren Additionssalze mit einer pharmazeutisch annehmbaren Säure,

**dadurch gekennzeichnet**, daß man ein Derivat der Formel (II)

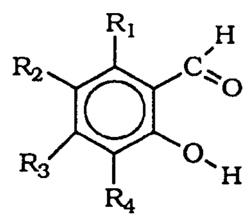

(II)

worin $R_I$, $R_2$, $R_3$ und $R_4$ die bezüglich der allgemeinen Formel (I) angegebenen Bedeutungen besitzen, mit 3-Oxo-buten-l der Formel (III)

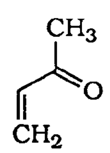

(III)

in Gegenwart eines alkalischen Mittels, welches vorzugsweise aus Alkaliund Erdalkali-Carbonaten oder organischen Basen, wie beispielsweise Triethylamin oder Pyridin, oder einer Mischung aus einem Alkalioder Erdalkali-Carbonat und einer organischen Base, ausgewählt ist, in einem polaren Lösungsmittel, welches vorzugsweise aus Butan-2-oron, Dimethylformamid, Aceton und Dimethylsulfoxid ausgewählt ist, durch Erhitzen zum Sieden am Rückfluß kondensiert,

so daß man nach Abkühlen und gegebenenfalls Verdampfen des Reaktionsmediums, Wiederaufnahme mit Wasser, welches gegebenenfalls mit einem alkalischen Mittel, wie Natriumhydroxid, versetzt worden ist, Extrahieren mit einem geeigneten organischen Lösungsmittel, wie Diethylether, Dichlormethan oder Chloroform, Waschen der organischen Phase, Verdampfen des Lösungsmittels und Reinigen des Rückstands durch Kristallisation oder Destillation oder Chromatographie über eine mit Siliciumoxid oder Aluminiumoxid gefüllte Säure, ein Derivat der Formel (IV)

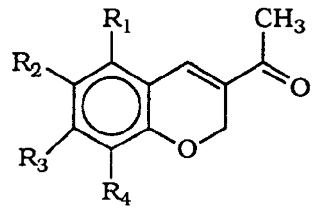

(IV)

in der $R_l$, $R_2$, $R_3$ und $R_4$ die bezüglich der Formel (I) angegebenen Bedeutungen besitzen, erhält,

welches nach dem Auflösen in einem geeigneten organischen Lösungsmittel, das vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung, vorzugsweise mit einem gemischten Alkalimetallhydrid, wie Natriumborhydrid, unterworfen wird, wonach man gegebenenfalls das Reaktionsmedium verdampft, mit einem geeigneten organischen Lösungsmittel, wie Diethylether, Methylenchlorid oder Chloroform, extrahiert, wäscht, die organische Phase vertungen besitzen, dampft und reinigt unter Bildung eines Derivats der Formel (V)

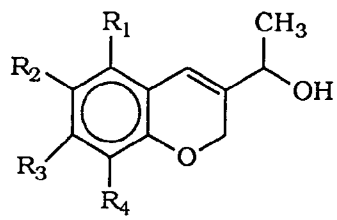

(V)

in der $R_l$, $R_2$, $R_3$ und $R_4$ die oben angegebenen Bedeutungen besitzen,

welches der Einwirkung eines Triphenylphosphoniumsalzes in einem Lösungsmittel, das vorzugsweise aus niedrigmolekularen aliphatischen Alkoholen ausgewählt ist, bei Raumtemperatur und unter Rühren unterworfen wird, so daß man nach dem Verdampfen des Lösungsmittels und Reinigung durch Chromatographie über Kieselgel oder durch Kristallisation aus einem geeigneten Lösungsmittel oder einer geeigneten Lösungsmittelmischung,

ein Derivat der Formel (VI)

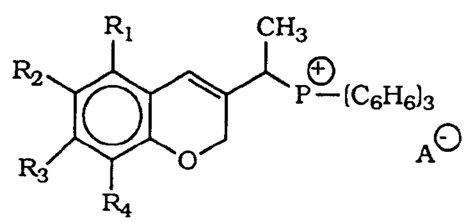

(VI)

erhält, in der $R_l$, $R_2$, $R_3$ und $R_4$ die bezüglich der Formel (I) angegebenen Bedeutungen besitzen und A das Anion einer Wasserstoffsäure bedeutet,

welches man nach der Suspension in einem organischen Lösungsmittel mit einer Lösung von n-Butyllithium in Hexan, vorzugsweise bei Raumtemperatur behandelt und dann mit einem Derivat der Formel (VII)

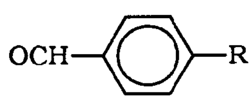

(VII)

in der R die bezüglich der Formel (I) angegebenen Bedeutungen besitzt,

vorzugsweise bei Raumtemperatur behandelt, so daß man nach dem Verdünnen mit Wasser oder durch Beseitigung des Lösungsmittels, Extraktion mit einem aus Diethylether, Diisopropylether, Chloroform und Methylenchlorid ausgewählten organischen Lösungsmittel und Reinigung durch Chromatographie über eine mit Siliciumoxid gefüllte Säule ein Derivat der Formel (I) erhält,

welches man gewünschtenfalls, wenn R eine Gruppe COOE, worin E für eine Niedrigalkylgruppe steht, einer alkalischen Hydrolyse unterziehen kann zur Bildung eines Derivats der Formel (I), worin R eine Gruppe COOH bedeutet,

welches man gewünschtenfalls

– entweder, wenn R eine Carboxylgruppe darstellt, mit einer pharmazeutisch annehmbaren Base in ein Salz überführen, oder, wenn R eine basische Gruppe aufweist, mit einer pharmazeutisch annehmbaren Säure in ein Salz umwandeln kann,

– oder mit Methoden der Kristallisation oder der Chromatographie in seine Isomeren, Diastereoisomeren oder Enantiomeren auftrennen und gewünschtenfalls, wenn R eine Carboxylgruppe bedeutet, mit einer pharmazeutisch annehmbaren Base in ein Salz überführen oder, wenn R eine basische Gruppe aufweist, mit einer pharmazeutisch annehmbaren Säure in ein Salz umwandeln kann.

2. Verfahren nach Anspruch I zur Herstellung der Derivate der Formel (I /A):

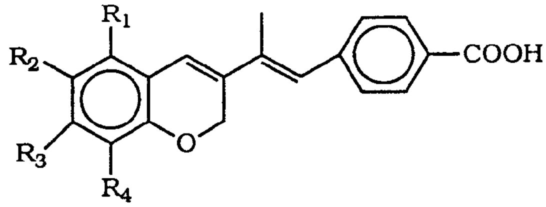

(I/A)

in der $R_I$, $R_2$, $R_3$ und $R_4$ die in Anspruch I bezüglich der Verbindungen der Formel (I) angegebenen Bedeutungen besitzen, **dadurch gekennzeichnet**, daß man ein Derivat der Formel (I/B)

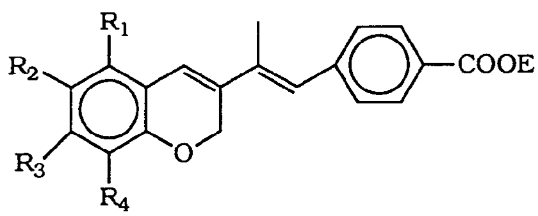

(I/B)

in der E eine Niedrigalkylgruppe darstellt und $R_I$, $R_2$, $R_3$ und $R_4$ die oben angegebenen Bedeutungen besitzen, einer alkalischen Hydrolyse unterwirft zur Bildung eines Derivats der Formel (I/A),

welches man gewünschtenfalls in seine Isomeren, Enantiomeren oder Diastereoisomeren auftrennen kann und

welches man gewünschtenfalls mit einer pharmazeutisch annehmbaren Base in ein Salz überführen kann.

3. Verfahren nach Anspruch I, zur Herstellung von Verbindungen der Formel (I), in der R eine Ethoxycarbonylgruppe bedeutet.

4. Verfahren nach Anspruch I, zur Herstellung von Derivaten der Formel (I), in der R eine Methoxycarbonylgruppe bedeutet.

5. Verfahren nach Anspruch I, zur Herstellung von Derivaten der Formel (I), in der R eine Niedrigalkylsulfonylgruppe bedeutet.

6. Verfahren nach Anspruch I, zur Herstellung von Derivaten der Formel (I), in der R eine Niedrigalkylaminocarbonylgruppe bedeutet.

7. Verfahren nach Anspruch I, zur Herstellung von Derivaten der Formel (I), in der R ein Wasserstoffatom bedeutet.

**8.** Verfahren nach Anspruch l, zur Herstellung von Derivaten der Formel (I), welche in der konfiguration (E) vorliegen.

**9.** Verfahren nach einem der Ansprüche l, 7 und 8 zur Herstellung des Derivats der Formel (I), nämlich 3-(l-Phenyl-2-propenyl)-2H-chromen (E).

**10.** Verfahren nach einem der Ansprüche l, 6 und 8 zur Herstellung des Derivats der Formel (I), nämlich 3-[l-(4-Ethylaminocarbonyl-phenyl)-2-propenyl]-2H-chromen (E).

**11.** Verfahren nach einem der Ansprüche l, 5 und 8 zur Herstellung des Derivats der Formel (I), nämlich 3-[l-(4-Ethylsulfonyl-phenyl)-2-propenyl§-2H-chromen (E).

**12.** Verfahren nach einem der Ansprüche l, 5 und 8 zur Herstellung des Derivats der Formel (I), nämlich 3-[l-(4-Ethylsulfonyl-phenyl)-2-propenyl]-6,8-dimethyl-2H-chromen.

**13.** Verfahren nach einem der Ansprüche l, 5 und 8 zur Herstellung des Derivats der Formel (I), nämlich 3-[l-(4-Ethylsulfonyl-phenyl)-2-propenyl]-6-chlor-8-methyl-2H-chromen.